# EUROPEAN PATENT SPECIFICATION

(11) **EP 3 079 480 B1**
(45) Date of publication and mention of the grant of the patent: **08.05.2019**
(21) Application number: 14821401.8
(22) Date of filing: 09.12.2014
(51) Int. Cl.: A23C 19/06, C12R 1/46, A23C 19/11

(54) **PROCESSED CHEESE WITH NATURAL ANTIBACTERIAL AND ANTIMYCOTIC COMPONENTS AND METHOD OF MANUFACTURING**
KÄSEPRODUKT ENTHALTEND NATÜRLICHE ANTIBAKTERIELLE UND ANTIMYKOTIKA, UND HERSTELLUNGSVERFAHREN
PRODUIT FROMAGER COMPRENANT DES ANTIBACTERIENS ET ANTIMYCOTIQUES NATURELS, ET METHODE DE PRODUCTION

(30) Priority: 10.12.2013 US 201361914246 P
(43) Date of publication of application: 19.10.2016
(73) Proprietor: Kraft Foods Group Brands LLC, Northfield, IL 60093 (US)
(72) Inventor: MARCUS-JOHNSON, Christine D., Chicago, Illinois 60626 (US); CHINWALLA, Ammar N., Northbrook, IL 60062 (US); REEVE, Jon L., Glenview, Illinois 60025 (US)
(74) Representative: Gill Jennings & Every LLP
(86) International application number: PCT/US2014/069278
(87) International publication number: WO 2015/089029

(56) References cited:
- WO-A1-2014/031842
- CN-A- 102 715 249
- US-A- 4 584 199
- US-A- 5 895 680
- US-A- 6 110 509
- US-A1- 2011 053 832
- QIAO M ET AL: "Regulation of the nisin operons in Lactococcus lactis N8", JOURNAL OF APPLIED BACTERIOLOGY, BLACKWELL PUBLISHING LTD., OXFORD, GB, vol. 80, no. 6, 1 January 1996 (1996-01-01), pages 626-634, XP002659299, ISSN: 0021-8847

### SEQUENCE LISTING

<110> Kraft Foods Group Brands LLC
<120> PROCESSED CHEESE WITH NATURAL ANTIBACTERIAL AND ANTIMYCOTIC
   COMPONENTS AND METHOD OF MANUFACTURING
<130> PJF11460EP
<140> EP 14821401.8
   <141> 2014-12-09
<150> PCT/US2014/069278
   <151> 2014-12-09
<150> US 61/914246
   <151> 2013-12-10
<160> 33
<170> PatentIn version 3.5
<210> 1
   <211> 34
   <212> PRT
   <213> Lactococcus lactis
<220>
   <221> MISC_FEATURE
   <222> (2)..(2)
   <223> Dehydrobutyrine (beta-methyldehydroalanine)
<220>
   <221> THIOETH
   <222> (3)..(7)
<220>
   <221> MISC_FEATURE
   <222> (5)..(5)
   <223> Dehydroalanine
<220>
   <221> THIOETH
   <222> (8)..(11)
<220>
   <221> MISC_FEATURE
   <222> (8)..(8)
   <223> Amino butyric acid
<220>
   <221> THIOETH
   <222> (13)..(19)
<220>
   <221> MISC_FEATURE
   <222> (13)..(13)
   <223> Amino butyric acid
<220>
   <221> THIOETH
   <222> (23)..(26)
<220>
   <221> MISC_FEATURE
   <222> (23)..(23)
   <223> Amino butyric acid
<220>
   <221> THIOETH
   <222> (25)..(28)
<220>
   <221> MISC_FEATURE
   <222> (25)..(25)
   <223> Amino butyric acid
<220>
   <221> MISC_FEATURE
   <222> (33)..(33)
   <223> Dehydroalanine
<400> 1
   Ile Xaa Ala Ile Xaa Leu Ala Xaa Pro Gly Ala Lys Xaa Gly Ala Leu
   1               5                   10                  15
   Met Gly Ala Asn Met Lys Xaa Ala Xaa Ala His Ala Ser Ile His Val
               20                  25                  30
   Xaa Lys
<210> 2
   <211> 1086
   <212> DNA
   <213> Lactococcus lactis
<400> 2
   atgaaacaga aacataaatt agcgcttggt gcgtcaattg ttgctttggc aagtcttggt     60
   gggattaaag cacaagctgc atctgttcaa gaaattatta acgctgcggt accagtggca    120
   aatgactacg gactttatcc atcagtaatg attgcccaag ggattttaga atcaagtggt    180
   ggacaaagtg ccttagcaag caattataat aatatttttg gagttaaata cacttctggt    240
   acacctgttt atctaccaac acaagagtat ttgaatggaa caatgacaaa tgttgttgaa    300
   cccttccaag cttatagctc agtttatgac gcatgtgttg cccaagctaa aatgttacgt    360
   gcttcatcat attattctgg ggcttggcgt gaaaatacaa gttcttactt agatgcgaca    420
   gcttggcttg aaggacgtta tgccacggat ccaacttatg cttctaaatt gaatagcgtg    480
   atttctgaac ttggtttaag tgtttatgac caaggaggag aaatatcagg aggaactgct    540
   gttacaacta gttcatcagc ctcaacaaat tcagctggca catacaaagt acaagagggt    600
   gattcattat cagcaatcgc tgctcaatat ggtacaactg ttgatgcact tgtgtcagca    660
   aatagtttag aaaatgcgaa cgatattcat gtaggagaag ttttgcaagt tgctggtgct    720
   agcacaacta caacaagtac caatacaact tccaatgtat cgtcaagttc tacttatacc    780
   gtcaaatcag gagatagttt atattcgatt gcggaacaat atggaatgac tgtttcatca    840
   ctgatgtcag ccaatggaat ttatgatgtt aattcaatgc ttcaagtagg acaagtattg    900
   caagtaactg taagtactag tgcaacaact tcaaacacaa cgacttcaaa cagttataca    960
   attcaaaatg gtgacagcat ttattcaatt gccacagcaa atggtatgac agctgaccaa   1020
   ttagcagccc tcaatggatt tggaattaat gacatgattc atccaggaca aacaattaga   1080
   atctaa                                                              1086
<210> 3
   <211> 1011
   <212> DNA
   <213> Lactococcus lactis
<400> 3
   atggtagtta aagttggtat taacggtttc ggtcgtatcg gtcgtcttgc tttccgtcgt      60
   attcaaaatg ttgaaggtgt tgaagttgtt gcaatcaacg acttgacaga tccagcaatg     120
   cttgctcact tgcttaaata cgatacaact caaggtcgtt ttgatggtaa agttgaagtt     180
   aaagatggtg gttttgaagt taacggtaaa ttcgttaaag ttactgctga atctaaccca     240
   gctaacatca actgggctga agttggtgca gaaatcgttc ttgaagcaac tggtttcttc     300
   gcaactaaag aaaaagctga acaacacttg cacgctaacg gtgctaaaaa agttgttatc     360
   actgcacctg gtggatctga tgttaaaaca atcgttttca acactaacca cgaagtactt     420
   gatggaactg aaacagtaat ttcagctggt tcatgtacaa ctaactgtct tgctccaatg     480
   gctgatactt tgaacaaaca attcggtatc aaagttggta caatgactac agttcacggt     540
   tacactggtg accaaatgac tcttgatggc ccacaccgtg gtggagactt ccgtcgcgca     600
   cgtgctgcag ctgaaaacat cgtacctaac tcaacaggtg ctgctaaagc tatcggtctt     660
   gtattgccag aacttcaagg taaacttcaa ggacatgctc aacgtgtacc agttccaact     720
   ggttcattga ctgaacttgt tactatcctt aacaaagaag ttacagttga cgaaatcaac     780
   gcagctatga aagctgcttc aaatgaatca tttggttaca acgaagacca aatcgtttca     840
   tctgatatcg ttggtatctc aaactcttca ctctttgatg ctactcaaac tgaagttact     900
   tcagctgatg gagctcaact tgttaaaact gtatcttggt acgataacga aatgtcatac     960
   acttcaaacc ttgttcgtac acttgcatac ttcgctaaaa tcgctaaata a             1011
<210> 4
   <211> 1419
   <212> DNA
   <213> Lactococcus lactis
<400> 4
   atggttgttg gtgcacaagc aacagaagtt gatttggttg ttattggttc aggccctggt      60
   ggttatgttg cagccatccg tgcggctgaa cttggtaaaa aagttacaat tattgaaaaa     120
   gataatgttg gtggggtttg tttaaatatt ggttgtatcc catcaaaagc attgattaat     180
   attggtcatc attaccaaga atctttggag gaagaaaaag gagaaaatcc ttttggtctt     240
   tctgtcggaa atgttaaatt aaactgggaa tctgcccaaa aatggaaaca agataaagtt     300
   gtcaaccagt tgacaggtgg tgttaaaatg ctacttaaaa aacacaaagt tgacgtgatt     360
   caaggaactg cagaatttat tgataacaat acaataaatg ttgaacaaga agatgggttc     420
   caacttttgc aatttaatga tgtgattatc tcaactggtt cacgtcctat cgaaattcct     480
   tctttcccat ttggtggtcg cattattgac tctactggtg ctttgtcact tccagaagtt     540
   cctaaacatt tgattattgt tgggggagga gttattggtt ctgagcttgg tggagcatac     600
   cgtatgctcg gttctaagat tacaattgtt gaaggtttgg accacatttt aaacgggttt     660
   gataaagaaa tgtctgatat cattgctaat cgcgttaaat ctgctggttc tgaaatcttt     720
   acttcagcaa tggctaaatc agctactcaa actgataaag atgtaacttt gacttttgag     780
   gttgacggaa aagaacaaac ggtgactggt gattacttac tcgtttctgt tggacgtcgt     840
   ccaaatactg atttaatcgg cttgaacaac actgatgtta aattgactga ccgtggtttg     900
   attgaagttg acgattctta tgcaactaat gttcctcaca tttatgcaat cggtgatgtg     960
   gttcctggtc caatgctcgc tcacaaagct tctttccaag ctaaagttgc tgctgctgcg    1020
   attgctggag ctgaggacga cgtggactta cacgttgctt tgcctgctgt agcttataca    1080
   acaactgaat tagcaacagt tggagaaacg cctgaatcag ttaaagaccg taaagatgtt    1140
   aaaatttcta agttcccatt tgctgcaaat ggccgtgcca tttcaatgaa tgatacgact    1200
   ggtttcttac gtttgattac tgaaactaaa gaaggggcct taatcggtgc tcaaatcgtt    1260
   ggccctggtg catctgactt gatttctggt ttatcactag cgattgaaaa tggattgact    1320
   tctaaagaca tttcattgac tatccaacct cacccaacac ttggtgaagc gattatggat    1380
   acagctgaat tggctgatgg cttaccaatt cacgtttaa                           1419
<210> 5
   <211> 1311
   <212> DNA
   <213> Lactococcus lactis
<400> 5
   atgacagtaa catcagattt cacacaaaaa ctctacgaaa attttgcaga aaatacaaaa      60
   ttgcgtgcgg tggaaaatgc cgtgactaaa aatggtttgc tttcatcact cgaagtccgt     120
   ggttcacatg cagcaaattt gcctgagttt tcaattgact tgacaaaaga ccctgtaacg     180
   aatcaaaaac aatctggtcg ttgctggatg tttgctgctt tgaacacttt ccgtcataaa     240
   tttatcaatg aatttaaaac agaggatttt gagttttcac aagcttacac tttcttctgg     300
   gataaatatg aaaaatcaaa ctggttcatg gaacaaatta ttggtgatat tgaaatggac     360
   gatcgtcgtt tgaaattcct tttacaaaca ccacaacaag atggcggcca atgggatatg     420
   atggttgcaa tttttgaaaa atatggaatt gttcccaaag ctgtttatcc tgaatcacaa     480
   gcttcaagta gctcacgtga attgaatcaa tacttgaata aactactccg tcaagatgct     540
   gaaattttgc gttatacaat tgagcaaggt ggagatgttg aagcagttaa agaagaactt     600
   ttgcaagaag tctttaattt ccttgcggta actttaggtt tgccaccaca aaattttgaa     660
   tttgctttcc gtaataaaga taatgaatac aaaaaatttg ttggtagtcc aaaagaattt     720
   tacaatgaat atgttggaat tgatttgaat aattatgtgt cagtaatcaa tgctccaact     780
   gctgacaaac cttataataa gagctacaca gttgagtttc ttggaaatgt tgtcggtggt     840
   aaagaagtga aacatttgaa tgttgaaatg gaccgcttta aaaaattggc cattgcccaa     900
   atgcaagctg gtgaaacagt ttggtttggt tgtgacgtgg gtcaagaatc aaatcgttca     960
   gcaggacttt tgacaatgga ttcttatgat ttcaaatctt cattggatat tgaatttact    1020
   caaagcaaag caggacgtct tgactatggt gagtcgttga tgacgcatgc catggtttta    1080
   gcgggtgttg atttagatgc tgacggaaat tcaactaaat ggaaagttga aaattcatgg    1140
   ggtaaagatg cgggtcaaaa aggatatttt gttgcctctg atgaatggat ggatgaatat    1200
   acttatcaaa ttgttgtccg taaagacctt ttaactgaag aagaattggc tgcttacgaa    1260
   gagaaacctc aagtacttct accatgggac ccaatgggtg ctttagctta a             1311
<210> 6
   <211> 648
   <212> DNA
   <213> Lactococcus lactis
<400> 6
   atgaaaaata aaattttaga cctgagggca tattttattg ctggcccaca agattttcca      60
   aaactttcaa ttgatgatgc aatcgataaa atttctgtaa tcataaaaag tggagtaacg     120
   gtttatcaat ttcgtgataa gggaacaatt tataaaaata aaaatcaacg attagaagta     180
   gcaaaaagac tacaagaagt agctcagaaa gcggctgttt cttttattgt gaatgatgat     240
   gttgaattag cgcgtgaatt gtcagctgac gggattcatg tcggacaaga cgatgattct     300
   gtcagtaaaa ttcgtgagct gattggccaa gaaatgtggg taggactttc tgtcagtaat     360
   gatatggaat tagaaagcgc tcaaaagagt ggggctgact atttgggaat tggtccaatt     420
   tatccaacaa atagcaagtc cgacgcagca gaaccaattg gggttgacca tttaagaaaa      480
   atgcttgagc ataatcaatt accaactgtt ggaattggtg gaattactga aaattcactg     540
   acagagcttt caaaaattgg tctgggtgga gttgcggtaa tttctttgct gacagaatcc     600
   gaaaattaca aaaatatggt tcaaaaaatt aagcaaaata ttagatga                  648
<210> 7
   <211> 1623
   <212> DNA
   <213> Lactococcus lactis
<400> 7
   atgaaacaac cactttataa tacaggagtt ttatttaaga ctttaataaa aagggattgg      60
   tttaagttag ttttctggat tttaggaatg cttgcttttg ctgcttcagg ggcagggaaa     120
   atggaagttg cctcgaatcc ggcgacggct agtactcttt atacaatgtt tgtcaaaaat     180
   ccagcaatgg tcggattatt tggaccaact ccaataaata atccaactaa ttatagtctg     240
   gggccgattt ttggtcaaac catgacttta attacggggc tgactttcgc tatcatttcg     300
   attatttatg ttgttaatcg aagcagaaaa gaagaagatg atgggattac agaacttttt     360
   cggtcttact ctattggaaa attggcaaat acgactgctt tagtcatgga acttttgctc     420
   ttaaatttaa taatggctgt cttattagct ctttcaatag aggtccaaaa cgtggctggc     480
   ttgaatcatt tagaaagtaa ttttctattt gctttcacaa caagcgctca gggtttcctt     540
   tggggaatgt ttgctttact tttcggtcaa attttctctg aagcaagtac aactaaaggg     600
   atgacatttg gtttactggg tttgttatat attgttcgaa tgctaacaga tgtaacaaat     660
   ctttccatag gttggttcaa tcctctgtct tggtcttatc tagcttttcc atatgttaaa     720
   ggtcatgaaa attggttagc tgtctttttg acttttctct tagcttttct aattttagga     780
   atatcctata ttctagagct taaaagagat gtgggagtgg ggtattttcc cgaaagaaag     840
   gcgcgacttc atgggaaaaa gggacatttc ggatttcctg gtctcgtttt gaatcttgaa     900
   aaaaagatga ttatcggttg gcttttggca agttttgttc tgggcttagt ttatggttca     960
   atgtttggac aaatggacca atttatttca agtaataaaa ccgttaagga gctttttgtt    1020
   gggaatgaaa cggcagcgag tgcgattaga ggaaacttca tggtcactct gttttcgata    1080
   ttgtcaatct taatcgcagc gtttggtgta attttactga caaaaatggt gagcgaggaa    1140
   agaaaaaatc gtctggaagc tctttatgct ttaccacttt cacgactaaa agtgtattcc    1200
   acttatttac tgatagctat tctgtcagta attttagctc agtttttagc gctttttgga    1260
   atatttattg aacagttggg taataaaaat gctttgagct tcttagaaat tatgaaatct    1320
   ggcatgattt ggcttgttgc tgtcatattt gttttagcaa tacttagtct gttacttggg    1380
   cttgtgcctc gtttggcaga attaatttgg gtatatcttg ctttcttact ttttatgact    1440
   tatcttggaa aattattatc tttgccaaaa tggcttgaaa atttaagcat ttataactat    1500
   attcctaaat tgccagttga gaaaatgaat cttcctaccg ttttattcat attaatttta    1560
   tctgtcttct tagttttact tggctttgga gcttatagaa gacgcgattt aatcacgggg    1620
   taa                                                                  1623
<210> 8
   <211> 1116
   <212> DNA
   <213> Lactococcus lactis
<400> 8
   atggctttaa cagcaggtat cgttggttta ccaaacgttg gtaaatcaac tctttttaat      60
   gcaattacaa aagcaggcgc agaagctgca aactatcctt ttgcaacaat tgacccaaat     120
   gttgggatgg tagaagtacc ggatgaacgt ttaaacaagt taacagagtt gattaaacct     180
   aagaaaactg ttccgacaac ttttgaattt acagatattg ctgggattgt taaaggggct     240
   tcacgcggag aaggtctagg aaataaattt cttgccaata ttcgtgaagt agatgccatc     300
   attcacgttg ttcgagcttt tgatgatgaa aatgtaatgc gtgaaaataa tcgtgaagat     360
   gcttttattg atccaatggc agacattgaa acaattaatc ttgaattaat tttggccgat     420
   ttagaatcag tcaataaacg ttatgcgcgt gttgaaaaag ttgctcgtac ggcaaaagat     480
   aaagatgcgg tcgcagaatt taatgttttg aaaaagctca aaccggtact tgaagatggt     540
   aaatcagcac gaacaattga ctttgacgaa gatgaaataa aggttcttaa aagcttgttc     600
   ttattgacaa gtaaaccagt tctttatgta gctaatgttt cagaagatga agtaggcgaa     660
   cctgataata tcgaatacgt gaaacaaatt cgtgagtttg cggcgactga aaatgctgaa     720
   gttgctgtga tttctgctcg tgttgaagaa gaaatctcag agttggaaga tgatgaaaaa     780
   gcagaatttt tggaagcaat tggcttaaaa gaatctggtg ttgatatgtt gactcgtgca     840
   gcttaccacc ttcttggact tgccacttac tttactgctg gtgaaaaaga agtccgtgct     900
   tggaccttca agcgtggaat gaaagctcca caaatggcag gaattattca tacagacttt     960
   gaaaaaggct ttatccgcgc agtaactatg tcttatgatg atttgcttaa atacggttca    1020
   gaaaaagctg ttcgtgaagc cggtcgcttg cgtgaagaag gaaaagaata tgttggtcaa    1080
   gatggcgaca ttatggaatt ccgtttcaac gtgtaa                              1116
<210> 9
   <211> 411
   <212> DNA
   <213> Lactococcus lactis
<400> 9
   atggatcgtt atactaaaaa agtcattgct tgggatttag gaaagcgaat gactctagaa      60
   ttagtgcaaa ggactttgaa taaggcaatg gaatcacaaa attatccaga agctgtgatg     120
   cttcattctg accaaggaag tcagtatacg agtcatgagt atgaagagac aataaaaaac     180
   tctggaatga ctcactcctt cagtcgtaag ggctatcctt atcataatgc cagtcttgaa     240
   tcttggcatg gacatttaaa aagagagtgg gtgtatcaat ttaaatataa gaactttgaa     300
   gaagcctatc agagtatttt ctggtacatc gaagcctttt ataattcaaa acgaatccat      360
   caaagtttag ggtatcttac gcctaatcaa tttgaaaaag aaatcactta a               411
<210> 10
   <211> 645
   <212> DNA
   <213> Lactococcus lactis
<400> 10
   atgataagaa gtgaatgtct caaattaaaa aatagcttag ggttttattt agtttttctc       60
   tttactttat tagagctttt aacggttcct atttatttag cttttggaag aagtcatgtt      120
   tcaatgactg atttatcgct catgattttt ttgttttttc cgttactggt tacaattttg      180
   tctattctaa tctttgaaca ggagagtctg gccaatcgtt tccaagaaat aaatgtaaat      240
   aaaaaaagta gcagaatttg gttatcaaag ctaatagtag tggatttcct tttgttcttt      300
   ccatcagcaa tgatctggat aattacggga gtttcacagg cagtagggca acaaggaatg      360
   atgatcgcaa cagctagctg gttgatggca atttttctta atcattttca tcttttattg      420
   acctttataa tcaatcgagg agggagcatg attatcgcga ttattgaaat attactcatt      480
   atttttgcca gtaataaagt tttattagca gcttattggt gtcccattgc tttacctgtt      540
   aattttatga taactgggcg gtgtgcttat ctgatagctg ccgtagggtg gattgtttta      600
   tccacaataa ttcttgtagc attatctaaa aaaaagatta gataa                      645
<210> 11
   <211> 729
   <212> DNA
   <213> Lactococcus lactis
<400> 11
   atgaaaagaa taatagcatc agaagcaata aaattaaaaa aatcaggaac tcttagattg        60
   gtattaatta tcccttttgt gactctattt atagcatttc ttatgggtgg aatacagatt        120
   tttagtgttt tttcaattta ttggtgggaa actggttttt tattcctttt gatgagtttg        180
   ctttttcttt atgatataaa atcagaggag caagctggaa attttcaaaa tgtgaaatgg        240
   aaaaagctga gttggaaaat tcatttggcc aaaatgttgt tgatttggct aagaggtata        300
   ctagcgagca tagtcttgat tattttgctt tatttggttg cttttgtgtt tcaaggtatt        360
   gtagtggtgg attttatgaa agtaagtgtg gcattgattg ctatattact agcagcttct        420
   tggaatttac cctttatata cttgattttc aagtggatta atacttacgt attgttagct        480
   gcgaatacct tgatttgttt aattgttgcc ccttttgttg cacaaactcc agtatggttc        540
   ttgctaccat acacttatca ctataaagtt acagaaagtt tgttaaatat caaaccatca        600
   ggagatttgt taacagggaa gataaatttc agtatttggg aagttttatt accatttgga        660
   ctttccatag ttgtaacgat aggagtttcg tatttactta aaggagtgat agaacatgat        720
   aagaagtga                                                               729
<210> 12
   <211> 678
   <212> DNA
   <213> Lactococcus lactis
<400> 12
   atgcaggtaa aaattcaaaa tctttctaaa acatataaag aaaagcaggt gctacaagat        60
   atcagttttg atattaaatc tggaacagtc tgtggtttat taggagttaa cggtgcagga        120
   aaatcaactt tgatgaaaat tttgtttggt ttaatttctg cagatactgg aaaaattttt        180
   tttgatggac aagaaaagac aaataatcaa cttggagcct taatcgaggc tccagcaata        240
   tatatgaatt tatctgcttt cgataatctt aaaactaagg ctttgctttt tggaatttca        300
   gataagagaa ttcatgaaac tctagaagtg attggtttgg cagaaacagg aaagaaaaga        360
   gcaggaaaat tctctttagg gatgaaacaa cgtttgggaa ttggtatggc tattcttaca        420
   gaacctcaat ttttaattct tgatgaacct actaatggtt tggatcctga tggtattgcg        480
   gagttgttaa acttaatctt aaaacttaaa gctaaaggtg tgacaatctt gatttctagt        540
   catcagttgc acgaaataag taaagtagct agtcaaatta ttattttgaa caaaggtaag        600
   attcgttata atcgtgcgaa caataaagaa gacgacattg aacagttatt ctttaagatt        660
   gtgcatggag gaatgtga                                                     678
<210> 13
   <211> 687
   <212> DNA
   <213> Lactococcus lactis
<400> 13
   gtgtataaaa ttttaatagt tgatgatgat caggaaattt taaaattaat gaagacagca      60
   ttagaaatga gaaactatga agttgcgacg catcaaaaca tttcacttcc cttggatatt     120
   actgattttc agggatttga tttgattttg ttagatatca tgatgtcaaa tattgaaggg     180
   acagaaattt gtaaaaggat tcgcagagaa atatcaactc caattatctt tgttagtgcg     240
   aaagatacag aagaggatat tataaacggc ttaggtattg gtggggatga ctatattact     300
   aagcctttta gccttaaaca gttggttgca aaagtggaag caaatataaa gcgagaggaa     360
   cgcaataaac atgcagttca tgttttttca gagattcgta gagatttagg accaattaca      420
   ttttatttag aagaaaggcg agtctgtgtc aatggtcaaa caattccact gacttgtcgt     480
   gaatacgata ttcttgaatt actatcacaa cgaacttcta aagtttatac gagagaggat     540
   atttatgatg acgtatatga tgaatattct aatgcacttt ttcggtcaat ctcggagtat     600
   atttatcaga ttaggagtaa gtttgcacca tacgatatta atccgataaa aacggttcgg     660
   ggacttgggt atcagtggca tgggtaa                                         687
<210> 14
   <211> 1344
   <212> DNA
   <213> Lactococcus lactis
<400> 14
   atgggtaaaa aatattcaat gcgtcgacgg atatggcaag ctgtcattga aattatcata        60
   ggtacttgtc tacttatcct gttgttactg ggcttgactt tctttctacg acaaattgga       120
   caaatcagtg gttcagaaac tattcgttta tctttagatt cagataattt aactatttct       180
   gatatcgaac gtgatatgaa acactaccca tatgattata ttatttttga caatgataca       240
   agtaaaattt tgggaggaca ttatgtcaag tcggatgtac ctagttttgt agcttcaaaa       300
   cagtcttcac ataatattac agaaggagaa attacttata cttattcaag caataagcat       360
   ttttcagttg ttttaagaca aaacagtatg cctgaattta caaatcatac gcttcgttca       420
   atttcttata atcaatttac ttaccttttc ttttttcttg gtgaaataat actcattatt        480
   ttttctgtct atcatctcat tagagaattt tctaagaatt ttcaagccgt tcaaaagatt       540
   gcattgaaga tgggggaaat aactactttt cctgaacaag aggaatcaaa aattattgaa       600
   tttgatcagg ttctgaataa cttatattcg aaaagtaagg agttagcttt ccttattgaa       660
   gcggagcgtc atgaaaaaca tgatttatcc ttccaggttg ctgcactttc acatgatgtt       720
   aagacacctt taacagtatt aaaaggaaat attgaactgc tagagatgac tgaagtaaat       780
   gaacaacaag ctgattttat tgagtcaatg aaaaatagtt tgactgtttt tgacaagtat       840
   tttaacacaa tgattagtta tacaaaactt ttgaatgatg aaaatgatta caaagcgaca       900
   atctccctgg aggatttttt gatagattta tcagttgagt tggaagagtt gtcaacaact       960
   tatcaagtgg attatcagct agttaaaaaa acagatttaa ccacttttta cggaaataca      1020
   ttagctttaa gtcgagcact tatcaatatc tttgttaatg cctgtcagta tgctaaagag      1080
   ggtgaaaaaa tagtcagttt gagtatttat gatgatgaaa aatatctcta ttttgaaatc      1140
   tggaataatg gtcatccttt ttctgaacaa gcaaaaaaaa atgctggaaa actatttttc      1200
   acagaagata ctggacgtag tgggaaacac tatgggattg gactatcttt tgctcaaggt      1260
   gtagctttaa aacatcaagg aaacttaatt ctcagtaatc ctcaaaaagg tggggcagaa      1320
   gttatcctaa aaataaaaaa gtaa                                             1344
<210> 15
   <211> 2010
   <212> DNA
   <213> Lactococcus lactis
<400> 15
   ttgggtttat cagcaactgt gcatggggag acaacaaatt cacaacagtt actctcaaat      60
   aatattaata cggaattaat taatcataat tctaatgcaa ttttatcttc aacagaggga     120
   tcaacgactg attcgattaa tctaggggcg cagtcacctg cagtaaaatc gacaacaagg     180
   actgaattgg atgtaactgg tgctgctaaa actttattac agacatcagc tgttcaaaaa     240
   gaaatgaaag tttcgttgca agaaactcaa gttagttctg aattcagtaa gagagatagc     300
   gttacaaata aagaagcagt tccagtatct aaggatgagc tacttgagca aagtgaagta     360
   gtcgtttcaa catcatcgat tcaaaaaaat aaaatcctcg ataataagaa gaaaagagct     420
   aacttcgtta cttcctctcc gcttattaag gaaaaaccat caaattctaa agatgcatct     480
   ggtgtaattg ataattctgc ttctcctcta tcttatcgta aagctaagga agtggtatct     540
   cttagacaac ctttaaaaaa tcaaaaagta gaggcacaac ctctattgat aagtaattct     600
   tctgaaaaga aagcaagtgt ttatacaaat tcacatgatt tttgggatta tcagtgggat     660
   atgaaatatg tgacaaataa tggagaaagc tatgcgctct accagccctc aaagaaaatt     720
   tctgttggaa ttattgattc aggaatcatg gaagaacatc ctgatttgtc aaatagttta     780
   ggaaattatt ttaaaaatct tgttcctaag ggagggtttg ataatgaaga acctgatgaa     840
   actggaaatc caagtgatat tgtcgacaaa atgggacacg ggacggaagt cgcaggtcag     900
   attacagcaa atggtaatat tttaggagta gcaccaggga ttactgtaaa tatatacaga     960
   gtatttggtg aaaatctttc gaaatcggaa tgggtagcta gagcaataag aagagctgcg    1020
   gatgatggga acaaggtcat caatataagt gctggacagt atcttatgat ttcaggatcg    1080
   tatgatgatg gaacaaatga ttatcaagag tatcttaatt ataagtcagc aataaattat    1140
   gcaacagcaa aaggaagtat tgttgtcgca gctcttggta atgatagttt aaacatacaa    1200
   gataaccaaa caatgataaa ctttcttaag cgtttcagaa gtataaaggt tcctggaaaa    1260
   gttgtagatg caccgagtgt atttgaggat gtaatagccg taggtggaat agatggttat    1320
   ggtaatattt ctgattttag taatattgga gcggatgcaa tttatgctcc tgctggcaca    1380
   acggccaatt ttaaaaaata tgggcaagat aaatttgtca gtcagggtta ttatttgaaa    1440
   gattggcttt ttacaactac taatactggc tggtaccaat atgtttatgg caactcattt    1500
   gctactccta aagtatctgg ggcactggca ttagtagttg ataaatatgg aataaagaat    1560
   cctaaccaac taaaaaggtt tcttctaatg aattctccag aagttaatgg gaatagagta    1620
   ttgaatattg ttgatttatt gaatgggaaa aataaagctt ttagcttaga tacagataaa    1680
   ggtcaggatg atgctattaa acataaatcg atggagaatc ttaaagagtc tagggataca    1740
   atgaaacagg aacaagataa agaaattcaa agaaatacaa ataacaattt ttctatcaaa    1800
   aatgattttc ataacatttc aaaagaagta atttcagttg attataatat taatcaaaaa    1860
   atggctaata atcgaaattc gagaggtgct gtttctgtac gaagtcaaga aattttacct    1920
   gttactggag atggagaaga ttttttaccg gctttaggta tagtgtgtat ctcaatcctt 1980
   ggtatattga aaagaaagac taaaaattga 2010
<210> 16
   <211> 738
   <212> DNA
   <213> Lactococcus lactis
<400> 16
   atgagaagat atttaatact tattgtggcc ttaataggga taacaggttt atcagggtgt 60
   tatcaaacaa gtcataaaaa ggtgaggttt gacgaaggaa gttatactaa ttttatttat 120
   gataataaat cgtatttcgt aactgataag gagattcctc aggagaacgt taacaattcc 180
   aaagtaaaat tttataagct gttgattgtt gacatgaaaa gtgagaaact tttatcaagt 240
   agcaacaaaa atagtgtgac tttggtctta aataatattt atgaggcttc tgacaagtcg 300
   ctatgtatgg gtattaacga cagatactat aagatacttc cagaaagtga taagggggcg 360
   gtcaaagctt tgagattaca aaactttgat gtgacaagcg atatttctga tgataatttt 420
   gttattgata aaaatgattc acgaaaaatt gactatatgg gaaatattta cagtatatcg 480
   gacaccaccg tatctgatga agaattggga gaatatcagg atgttttagc tgaagtacgt 540
   gtgtttgatt cagttagtgg caaaagtatc ccgaggtctg aatgggggag aattgataag 600
   gatggttcaa attccaaaca gagtaggacg gaatgggatt atggcgaaat ccattctatt 660
   agaggaaaat ctcttactga agcatttgcc gttgagataa atgatgattt taagcttgca 720
   acgaaggtag gaaactag 738
<210> 17
   <211> 1245
   <212> DNA
   <213> Lactococcus lactis
<400> 17
   atgaataaaa aaaatataaa aagaaatgtt gaaaaaatta ttgctcaatg ggatgagaga       60
   actagaaaaa ataaagaaaa cttcgatttc ggagagttga ctctctctac aggattgcct      120
   ggtataattt taatgttagc ggagttaaaa aataaagata actcaaagat atatcagaaa      180
   aagatagaca attatattga atatattgtt agcaaacttt caacatatgg gcttttaaca      240
   ggatcacttt attcgggagc agctggcatt gcattaagta tcctacattt acgagaagat      300
   gacgaaaaat ataagaatct tcttgatagc ctaaatagat atatcgaata tttcgtcaga      360
   gaaaaaattg aaggatttaa tttggaaaac attactcctc ctgattatga cgtgattgaa      420
   ggtttatctg ggatactttc ctatctatta ttaatcaacg acgagcaata tgatgatttg      480
   aaaatactca ttatcaattt tttatcaaat ctgactaaag aaaacaatgg actaatatcg      540
   ctttacatca aatcggagaa tcagatgtct caatcagaaa gtgagatgta tccactaggc      600
   tgtttgaata tgggattagc acatggactt gctggagtgg gctgtatctt agcttatgcc      660
   cacataaaag gatatagtaa tgaagcctcg ttgtcagctt tgcaaaaaat tatttttatt      720
   tatgaaaagt ttgaacttga aaggaaaaaa cagtttctat ggaaagatgg acttgtagca      780
   gatgaattaa aaaaagagaa agtaattagg gaagcaagtt tcattagaga tgcatggtgc      840
   tatggaggtc caggtattag tctgctatac ttatacggag gattagcact ggataatgac      900
   tattttgtag ataaagcaga aaaaatatta gagtcagcta tgcaaaggaa acttggtatt      960
   gattcatata tgatttgcca tggctattct ggtttaatag aaatttgttc tttatttaag     1020
   cggctattaa atacaaaaaa gtttgattca tacatggaag aatttaatgt taatagtgag     1080
   caaattcttg aagaatacgg agatgaaagt ggcacgggtt ttcttgaagg aataagtggc     1140
   tgtatactgg tattatcgaa atttgaatat tcaatcaatt ttacttattg gagacaagca     1200
   cttttacttt ttgacgattt tttgaaagga gggaagagga aatga                     1245
<210> 18
   <211> 1803
   <212> DNA
   <213> Lactococcus lactis
<400> 18
   atggatgaag tgaaagaatt cacatcaaaa caatttttta atactttact tactcttcca      60
   agcaccttga agttaatttt tcagttggaa aaacgttatg caatttattt aattgtgcta     120
   aatgctatca cagcttttgt tccgttggct agtcttttta tttatcaaga tttaataaac     180
   tctgtgctag gttcagggag acatcttatc aatattatta tcatctattt tattgttcaa     240
   gtgataacaa cagttctggg acagctggaa agttatgtta gtggaaaatt tgatatgcga     300
   ctttcttaca gtatcaatat gcgcctcatg aggactacct catctcttga attaagtgat     360
   tatgagcagg ctgatatgta taatatcata gaaaaagtta ctcaagacag cacttacaag     420
   ccttttcagc tatttaatgc tatcattgtt gtgctttcat cgtttatctc attgttatct     480
   agtctatttt ttattggaac atggaacatt ggggtagcaa ttttactcct tattgttcca     540
   gtattatctt tggtactttt tctcagagtg ggacaattag agtttttaat ccagtggcag     600
   agagcaagtt ctgaaagaga aacatggtat attgtatatt tattgactca tgatttttca     660
   tttaaagaaa tcaagttaaa taatattagc aattacttca ttcataaatt tggaaaatta     720
   aagaaaggat ttatcaacca agatttagct attgctcgta agaagacata tttcaatatt     780
   tttcttgatt tcattttgaa tttgataaat attcttacga tatttgctat gatcctttcg     840
   gtaagagcag gaaaacttct tataggtaat ttggtaagtc tcatacaagc tatttctaaa     900
   atcaatactt attctcaaac aatgattcaa aatatttaca tcatttataa tactagtttg     960
   tttatggaac aactttttga gtttttaaag agagaaagtg tagttcacaa aaaaatagaa    1020
   gatactgaaa tatgcaatca acatatagga actgttaaag taattaattt atcatatgtt    1080
   taccctaatt cgaatgcctt tgcactaaag aatatcaatt tatcctttga aaaaggagaa    1140
   ttaactgcta ttgtaggaaa aaatggttca gggaaaagta cactagtaaa gataatttca    1200
   ggattatatc aaccaactat gggaataatc caatacgaca aaatgagaag tagtttgatg    1260
   cctgaggagt tttatcagaa aaacatatcg gtgctgttcc aagattttgt gaagtatgag    1320
   ttaacgataa gagagaatat aggattgagt gatttgtctt ctcaatggga agatgagaaa    1380
   attattaaag tactagataa tttaggactc gattttttga aaactaataa tcaatatgta    1440
   cttgatacgc agttaggaaa ttggtttcaa gaagggcatc aactttcagg aggtcagtgg    1500
   caaaaaattg cattagcaag gacattcttt aagaaagctt caatttatat tttagatgaa    1560
   ccaagtgctg cactcgatcc tgtagctgaa aaagaaatat ttgattattt tgttgctctt    1620
   tcggaaaata atatttcaat tttcatttct catagtttga atgctgccag aaaagcaaat    1680
   aaaatcgtgg ttatgaaaga tggacaggtc gaagatgttg gaagtcatga tgtccttctg    1740
   agaagatgtc aatactatca agaactttat tattcagagc aatatgagga taatgatgaa    1800
   taa                                                                  1803
<210> 19
   <211> 2982
   <212> DNA
   <213> Lactococcus lactis
<400> 19
   atgataaaaa gttcatttaa agctcaaccg tttttagtaa gaaatacaat tttatctcca      60
   aacgataaac ggagttttac tgaatatact caagtcattg agactgtaag taaaaataaa     120
   gtttttttgg aacagttact actagctaat cctaaactct atgatgttat gcagaaatat     180
   aatgctggtc tgttaaagaa gaaaagggtt aaaaaattat ttgaatctat ttacaagtat     240
   tataagagaa gttatttacg atcaactcca tttggattat ttagtgaaac ttcaattggt     300
   gttttttcga aaagttcaca gtacaagtta atgggaaaga ctacaaaggg tataagattg     360
   gatactcagt ggttgattcg cctagttcat aaaatggaag tagatttctc aaaaaagtta     420
   tcatttacta gaaataatgc aaattataag tttggagatc gagtttttca agtttatacc     480
   ataaatagta gtgagcttga agaagtaaat attaaatata cgaatgttta tcaaattatt     540
   tctgaatttt gtgagaatga ctatcaaaaa tatgaagata tttgtgaaac tgtaacgctt     600
   tgctatggag acgaatatag agaactatcg gaacaatatc ttggtagtct gatagttaat     660
   cattatttga tctctaattt acaaaaagat ttgttgtcag atttttcttg gaacactttt     720
   ttgactaaag ttgaagcaat agatgaagat aaaaaatata taattcctct gaaaaaagtt     780
   caaaagttta ttcaagaata ctcagaaata gaaattggtg aaggtattga gaaactgaaa 840
   gaaatatatc aggaaatgtc acaaattctt gagaatgata attatattca aattgattta 900
   attagtgata gtgaaataaa ttttgatgtt aaacaaaagc aacaattaga acatttagct 960
   gagtttttag gaaatacgac aaaatctgta agaagaacat atttggatga ctataaggat 1020
   aaatttatcg aaaaatatgg tgtagatcaa gaagtacaaa taacagaatt atttgattct 1080
   acatttggca taggagctcc atataattat aatcatcctc gaaatgactt ttatgagtcc 1140
   gaaccgagta ctctatacta ttcagaagag gagagagaaa agtacctcag catgtatgta 1200
   gaagccgtta aaaatcataa tgtaattaat cttgacgact tagagtctca ttatcaaaaa 1260
   atggacttag aaaagaaaag tgaacttcaa gggttagaat tatttttgaa tttggcaaag 1320
   gagtatgaaa aagatatttt tattttaggg gatatcgttg gaaataataa tttgggaggg 1380
   gcatcaggta gattttctgc actctctccg gagttaacaa gttatcatag aacgatagta 1440
   gattctgtcg aaagagaaaa tgagaataaa gaaattacat cgtgtgaaat agtatttctt 1500
   ccagaaaata tcagacatgc taacgttatg catacatcaa ttatgaggag gaaagtactt 1560
   ccatttttta caagtacaag tcacaatgaa gttctgttaa ctaatatcta tattggaata 1620
   gacgaaaaag aaaaatttta tgcacgagac atttcaactc aagaggtatt gaaattctac 1680
   attacaagca tgtacaataa aacgttattc agtaatgagc taagatttct ttacgaaatt 1740
   tcattagatg acaagtttgg taatttacct tgggaactta tttacagaga ctttgattat 1800
   attccacgtt tagtatttga cgaaatagta atatctcctg ctaaatggaa aatttgggga 1860
   agggatgtaa atagtaagat gacaataaga gaacttattc aaagcaaaga aattcccaaa 1920
   gagttttata ttgtcaatgg agataataaa gtttatttat cacaggaaaa cccattggat 1980
   atggaaattt tagagtcggc gataaagaag agctcaaaaa gaaaagattt tatagagcta 2040
   caagaatatt ttgaagatga aaatatcata aataaaggag aaaaggggag agttgccgat 2100
   gttgtagtgc cttttattag aacgagagca ttaggtaatg aagggagagc atttataaga 2160
   gagaaaagag tttcggttga acggcgtgaa aaattgccct ttaacgagtg gctttatcta 2220
   aagttgtaca tttctataaa tcgtcaaaat gaatttttac tgtcgtatct tccagatatt 2280
   cagaaaatag tagcaaacct gggtggaaat ctattcttcc taagatatac tgatcctaaa 2340
   ccacatatta gattgcgtat aaaatgttca gatttatttt tagcttacgg atctattctt 2400
   gaaatcttaa aaaggagtcg gaaaaatagg ataatgtcaa cttttgatat ttctatttat 2460
   gatcaagaag tagaaagata tggtggattt gatactttag agttatccga agcaatattt 2520
   tgtgccgatt ctaaaattat tccaaatttg cttacattga taaaagatac taataatgat 2580
   tggaaagtcg atgatgtatc aatcttggtg aattatttat atctgaaatg cttctttcag 2640
   aatgataaca aaaagattct taattttttg aatttagtta gtcctaaaaa ggttaaagaa 2700
   aatgtcaatg aaaagattga acattatctt aagcttctga aagttaataa tctaggtgac 2760
   caaatttttt atgacaagaa ttttaaagaa ttaaagcatg ccataaaaaa tttattttta 2820
   aaaatgatag ctcaagattt tgaacttcag aaagtttatt caattattga cagtatcatt 2880
   catgtccata ataaccgact aattggtatt gaacgagata aagagaaatt aatttattac 2940
   acacttcaaa ggttgtttgt ttcggaagaa tacatgaaat ga 2982
<210> 20
   <211> 57
   <212> PRT
   <213> Lactococcus lactis
<400> 20
   Met Ser Thr Lys Asp Phe Asn Leu Asp Leu Val Ser Val Ser Lys Lys
   1 5 10 15
   Asp Ser Gly Ala Ser Pro Arg Ile Thr Ser Ile Ser Leu Cys Thr Pro
   20 25 30
   Gly Cys Lys Thr Gly Ala Leu Met Gly Cys Asn Met Lys Thr Ala Thr
   35 40 45
   Cys His Cys Ser Ile His Val Ser Lys
   50 55
<210> 21
   <211> 318
   <212> DNA
   <213> Lactococcus lactis
<400> 21
   atgaatgatt tattttatca tcggctaaag gaactagttg aatcaagtgg taaatctgca 60
   aatcaaatag aaagggaatt gggttaccct agaaattctt tgaataatta taagttggga 120
   ggagaaccct ctgggacaag attaatagga ctatcagagt attttaatgt gtctccaaaa 180
   tatctgatgg gtataattga tgagcctaat gacagttctg caattaatct ttttaaaact 240
   ctaactcaag aagagaaaaa agaaatgttt ataatttgtc aaaaatggct ttttttagaa 300
   tatcaaatag agttataa 318
<210> 22
   <211> 768
   <212> DNA
   <213> Lactococcus lactis
<400> 22
   atgatgaaaa aaggaatttt tgtaattact atagtgatat ctatagcatt gataattgga      60
   ggtttttata gttataattc taggataaat aatctttcaa aagctgataa aggaaaagaa     120
   gttgtaaaaa atagcagtga aaaaaatcag atagacctta cctataaaaa gtattataaa     180
   aatttaccaa aatcagttca aaataaaata gatgatattt catccaaaaa taaagaagtt     240
   actttaactt gtatttggca atctgattca gttatttctg aacaatttca acaaaactta     300
   caaaaatatt atggaaataa gttttggaac atcaaaaata tcacttacaa tggcgaaact     360
   agtgaacaat tattggctga aaaagttgaa aaccaagtat tagccactaa tcctgatgtt     420
   gttttatatg aagctccact ttttaatgat aaccaaaaca ttgaagcaac agcctcactg     480
   actagtaatg agcaacttat aacaaatttg gctagtgcag gagcggaggt aatagttcaa     540
   ccctctccac cgatttatgg tggtgttgtg taccccgtac aagaagaaca gtttaaacaa     600
   tctttatcta caaagtatcc ctatatagac tactgggcta gttacccaga caaaaattct     660
   gatgaaatga aggggctgtt ttctgatgat ggagtatata gaacattaaa tgcttcgggg     720
   aataaggttt ggctagatta tattactaaa tattttacag caaactaa                  768
<210> 23
   <211> 780
   <212> DNA
   <213> Lactococcus lactis
<400> 23
   atgcaggaaa cacaggaaca gacgattgat ttaagaggga tttttaaaat tattcgcaaa      60
   aggttaggtt taatattatt tagtgcttta atagtcacaa tattagggag catctacaca     120
   ttttttatag cctccccagt ttacacagcc tcaactcaac ttgtcgttaa actaccaaat     180
   tcggataatt cagcagccta cgctggacaa gtgaccggga atattcaaat ggcgaacaca     240
   attaaccaag ttattgttag tccagtcatt ttagataaag ttcaaagtaa tttaaatcta     300
   tctgatgact ctttccaaaa acaagttaca gcagcaaatc aaacaaattc acaagttatt     360
   acgcttactg ttaaatattc taatccttac attgcacaaa agattgcaga cgagactgct     420
   aaaatattta gttcagacgc accgaaacta ttgaatgtta ctaacgttaa tattctatcc     480
   aaagcaaaag ctcaaacaac accaattagt cctaaaccta aattgtattt agcgatatct     540
   gttatagtcg gactagtttt aggtttagcc attgctttat tgaaagaatt gtttgataac     600
   aaaattaata aagaagaaga tattgaagct ctggggctca cggttcttgg tgtaacaacc     660
   tatgctcaaa tgagtgattt taataagaat acaaataaaa atggcacgca atcgggaact     720
   aagtcaagtc cgcctagcga ccatgaagta aatagatcat caaaaaggaa taaaagatag     780
<210> 24
   <211> 696
   <212> DNA
   <213> Lactococcus lactis
<400> 24
   atggctaaaa ataaaagaag catagacaac aatcgttata ttattaccag tgtcaatcct      60
   caatcaccta tttctgaaca atatcgtacg attcgtacga ccattgattt taaaatggcg     120
   gatcaaggga ttaaaagttt tctagtaaca tcttcagaag cagctgcagg taaatcaacc     180
   gcgagtgcta atatagctgt tgcttttgca caacaaggta aaaaagtact tttaattgat     240
   ggtgatcttc gtaaaccgac tgttaacatt acttttaaag tacaaaatag agtagggtta     300
   accaatattt taatgcatca atcttcgatt gaagatgcca tacaagggac aagactttct     360
   gaaaatctta caataattac ctctggtcca attccaccta atccatcgga attattagca     420
   tctagtgcaa tgaagaattt gattgactct gtgtccgatt tctttgatgt tgttttgatt     480
   gatactccac ctctctctgc agttactgat gctcaaattt tgagtagtta tgtaggagga     540
   gtggttcttg ttgtacgtgc ctatgaaaca aaaaaagaga gtttagcaaa aacaaaaaaa     600
   atgctggaac aagttaatgc aaatatttta ggagttgttt tgcatggggt agactcttct     660
   gactcaccgt cgtattacta ctacggagta gagtaa                               696
<210> 25
   <211> 687
   <212> DNA
   <213> Lactococcus lactis
<400> 25
   atggaatttt ttgaggatgc ctcatcacct gaatcggaag agcctaagtt agtagaatta 60
   aaaaattttt cttatagaga gctaattata aaaagagcaa ttgatatcct aggaggatta 120
   gcaggttcag ttttatttct tattgcggct gcattgcttt atgtgcctta caaaatgagc 180
   tcaaaaaaag atcaagggcc aatgttctat aaacaaaaac ggtatggaaa aaacggtaaa 240
   attttttata ttttgaaatt tagaacaatg attcttaatg ccgagcagta tctagaactt 300
   aatccagatg ttaaagctgc ttaccatgcc aacggcaata agctagaaaa cgatccacgg 360
   gtaacgaaga ttggctcatt tataagacga cactcaattg atgaactgcc acaatttatc 420
   aatgttctta aaggggatat ggcattggtt ggcccaagac caattttgct ttttgaagcg 480
   aaagaatatg gggagcgcct ctcttactta ctcatgtgta aacctggaat tactggttat 540
   tggacaacac atggtcgaag taaagttctt tttcctcaac gagcagattt agaactctat 600
   tacctccagt accatagtac caaaaacgat atcaagcttc tagtactcac aattgtacaa 660
   agtattaacg gatcggacgc atattaa 687
<210> 26
   <211> 765
   <212> DNA
   <213> Lactococcus lactis
<400> 26
   atgattgata ttcattgcca tattttaccg ggtatagatg atggagctaa aacttctgga 60
   gatactctga caatgctgaa atcagcaatt gatgaaggga taacaactat cactgctact 120
   cctcatcata atcctcaatt taataatgaa tcaccactta ttttgaaaaa agttaaggaa 180
   gttcaaaata tcattgacga acatcaatta ccaattgaag ttttacccgg acaagaggtg 240
   agaatatatg gtgatttatt aaaagaattt tctgaaggaa agttactgac agcagcgggc 300
   acttcaagtt atatattgat tgaatttcca tcaaatcatg tgccagctta tgctaaagaa 360
   cttttttata atattcaatt ggagggactt caacctattt tggtccaccc tgagcgtaat 420
   agcggaatca ttgagaaccc tgatatatta tttgatttta ttgaacaagg agtactaagt 480
   cagataacag cttcaagtgt cactggtcat tttggtaaaa aaatacaaaa gctgtcattt 540
   aaaatgatag aaaaccatct tacgcatttt gttgcatcag atgcgcataa tgtgacgtca 600
   cgtgcattta agatgaagga agcgtttgaa attattgaag atagttatgg ttctgatgta 660
   tcacgaatgt ttcaaaataa tgcagagtca gtgattttaa acgaaagttt ttatcaagaa 720
   aaaccaacaa agatcaaaac aaagaaattt ttaggattat tttaa 765
<210> 27
   <211> 450
   <212> DNA
   <213> Lactococcus lactis
<400> 27
   atgaaaatag cattagtagg ttccagcggt ggccatttga cacacctgta tttgttaaaa     60
   aagttttggg aaaacgaaga tagattttgg gtcacatttg ataaagcaga tgcaaaatct    120
   atattgaaag aagaaagatt ttatccttgt tattatccca caaatagaaa tgtaaaaaac    180
   acgataaaaa ataccattct tgcatttaaa atacttagaa aagaaaaacc agatttgatt    240
   atttcgagtg gtgctgcggt agccgttcct tttttttggt taggtaaact attcggtgca    300
   aagacagtct atattgaaat atttgaccgg atcgataaac caaccttaac aggaaaatta    360
   gtttatccag ttactgataa gtttatagtt caatgggaag agttaaaaaa agtttaccct    420
   aaagcaatta atttaggagg aattttctaa                                      450
<210> 28
   <211> 507
   <212> DNA
   <213> Lactococcus lactis
<400> 28
   atgatttttg taacggttgg aactcacgaa caaccattta atcgactcat tcaaaaaatt     60
   gatgaacttg tacgcgatgg tgaaatcgaa gacgatgtat tcatgcaaat tgggtactca    120
   acttatgaac ccaaatatac taaatgggaa aaggttattg gatatgagac tatggaaaga    180
   tgtatgaatg aagcgagtac gattattact catggcggac catctaccta tatgcaagta    240
   ttacaactag gtaaaattcc gatagttgtt ccacggcaaa tgaaatttga tgagcatata    300
   aatgatcatc aaatttgggt aagtaaacag gttgtgaaaa agggatactc attgattttg    360
   tgcgaagatg ttgaagacat tctcgaaaat attattagct ccaaaatttc agatacctta    420
   caaaaaaatg taaatcacaa cactgaattc ataaaattat tcagtgctga aatttaccag    480
   ctatttataa aaagtgagaa gatatga                                         507
<210> 29
   <211> 987
   <212> DNA
   <213> Lactococcus lactis
<400> 29
   atggttaaat tttctataat tattccagta tataacttag aagattattt atatcgctgt        60
   ttagaatctg ttctcaatca agattataat gattttgaga ttatacttat taatgatggt       120
   tcagatgata attcacttaa tattatcgaa gaatttaaaa atcaatattc tagtaaaata       180
   aaagtaattt ctcaagtgaa tcaaggagta tcatcagcaa gaaataaggg acttcaagaa       240
   gctgaagggg agtatataat ttttatagat ggggatgact acattgatag taatcattta       300
   agtaatattc ttgaatatat aggaaaatca aaaaattcct ttattctaaa tagtctattt       360
   gtagagaccg gggaaactac ttgggttatt cccaaagcaa gtaagaatta tgattgtagt       420
   ttttatggaa ctcttatgaa tattttagat aatcatagat atcaaggctt tttgtttaat       480
   aaaatattta gtaattcagt aataaaaagt aatgaattga agtttaaaga aaatttatat       540
   tatgcggaag atacggagtt tgttattcga taccttttgg agttgcaaaa gagagaatcg       600
   gatttagttg ctaatataat taattctcca acatatcatt atgttcaaat aaaaagtagt       660
   gctactcacc aatttaatat cagacaattt tctctagtaa attctatgga ggaaattcaa       720
   tgtaatctag aaaaaatgaa atcaataaat aaagaagttt tgtatgtagt acaatcaaat       780
   ttaattcagt cagtattgaa aatgataaga ttatctagaa taaatggtgt agttaatgaa       840
   catttggaag attcattaga taaaatcgta acaaattcat gggataatat tgaaactatc       900
   tggaaatctc aaagaaagat ttactctaag gtatttttaa cactaagaat tattcaggaa       960
   aaagttaagg gtaagaaaat caaatag                                           987
<210> 30
   <211> 867
   <212> DNA
   <213> Lactococcus lactis
<400> 30
   gtgataatta tacctatctt aatttttatt acccttatag gagcaggggc ttatgcctta        60
   agagattcac ttattcctac tgaacatacg aaaacaaata gttcggatca accgaccaaa       120
   acttcggttt ctaacggtta cgtggagcaa aaaggtgaag aagctgctgt gggtagtata       180
   gcacttgtag atgacgctgg agtaccagaa tgggttaaag ttccctcaaa ggtaaattta 240
   gataaattta ctgatttatc tacgaataat atcactattt atcgaattaa caatccggaa 300
   gtcttaaaaa cagttaccga tcgtacggat caacggatga aaatgtcaga agttatagct 360
   aagtatccta atactttgat tatgaatgct tccgcttttg atatgcagac aggacaagta 420
   gctggatttc aaattaataa tggaaagttg attcaagact ggagtccagg tacaacgact 480
   cagtatgctt ttgttattaa caaagatggt tcgtgcaaaa tttatgattc aagtacacct 540
   gcttcaacta ttattaaaaa cggagggcaa caagcctatg attttggtac tgcaattatc 600
   cgtgatggta aaattcaacc aagtgatggc tcagtagatt ggaagatcca tatttttatt 660
   gcgaatgata aagataataa tctctatgct attttgagtg atacaaatgc aggttatgat 720
   aatataatga aatcagtgtc aaatttgaag ctccaaaata tgttattact tgatagtggt 780
   ggttcaagtc aactatctgt caatggtaaa acgattgttg ctagtcaaga tgatcgagcc 840
   gtaccggatt atattgtgat gaaataa 867
<210> 31
   <211> 903
   <212> DNA
   <213> Lactococcus lactis
<400> 31
   atgaatcaaa aaaagaggcg tcattatcgt aagaaaaaat acacagtact aaaagttatt 60
   tcaattattt ttgtattagt aattatttct gttgcttcta tagcctacgt agcttataga 120
   aatgttgaat caaccttttc aacatcatat gaaaatttcc ctaaaacaac aagtattgac 180
   ttaaaaaaat ctaaaacatt caccacactt atcattgcaa ctggtaaaaa taattctaaa 240
   aattcagctt atgctactgt tttagcttca acgaatgtaa agacaaatca aactactttc 300
   atgaacttcc cagtttttgc gacactgcct aatcaaaaaa caatcactga agtttacaat 360
   acgaatggag atgatggaat tttccagatg gttaaagacc tattgaatgc gtccattaac 420
   aaagtaattc agatcgatgt taataaaatg ggatcacttg tacaggccac tggtggaatc 480
   accatgcaaa atccaaaggc attcaatgct gaaggttatg agtttaaaca aggaactgtt 540
   aatttacaaa ctgctgatca agtccaagcc tatatgacac aaattgacga tactgatttg 600
   gatgcttcaa tcactcggat tcaaaatgtc tcaatggaac tctacgtaaa tattaaaaaa 660
   attgctcata tgaaaaaact tgaaagtttc aattactatc gagaaattct ctatgctttt 720
   tcaaacactg ttaaaaccaa tataagtttc aatgatgcta aaacgatcgt tatgagctac 780
   agtaaggctc taaagaatac cagcaagctc aatctacata caacagatga aaatggagct 840
   aaggtcgttt ctcaaacaga attagactca gtcaaaaccc tttttgaaaa atctctaaaa 900
   taa 903
<210> 32
   <211> 1431
   <212> DNA
   <213> Lactococcus lactis
<400> 32
   ttgagtaaca caggaataaa agtaactaat aaccaaatac taataagaaa agtaacagtt      60
   atatttttgc ttttttcagg aatttgggta ggagctgcaa caagtttttt tgagatttct     120
   ttagtgtttt ctgtattagc tggtattata agctcaaatt tactagaaaa tgaatttgtt     180
   ccgtattcag ttattttaat actggttttt caaaatttat tgataggtac tgggtctcat     240
   ttaacaggga atctgtcagg tttaacttat ctcactcaag ttccgatggt ttttgtttgg      300
   actattaatc tctgtttgaa aaaggtagat aaattatcta aagttgatat ttcttttata     360
   gttcttatga tattttcttt gtcatctttg gcatttggac gagggccaat tcaggcaatt     420
   atttcgaact taagagattt atcgacattt tattttactt atcgtatagg taaaagattt     480
   atcaaaactg aagaaatatt tgcgaggttt ataaagaaaa ttctttattt aggaatattt     540
   gtagttttaa ttggtattat cttatactta ggaggctatc ctctaaacaa atttttggga     600
   atagatgaaa tttactacgc taagggggta actactttac ttaataattt tgatggcaga     660
   tttggatcag atgtatttgg aatttccgta acacgtatgg gatcaattta ttttgaacca     720
   ataaatttgg ggtatttaat attttctatg ctcattatat cctttatttt ctttaatacc     780
   caaaaattaa aatatataaa tttatataga ctgattttat tgataggtgg aatgttaact     840
   tttgggaaag gtgctatgct tctagctata ggggtgatgg ttgcaggtat cggacataaa     900
   ttatttttaa aattcttttc aagaagtaat gaaatgaatg tttttagaaa tctttttata     960
   ttgttaacta ttattatgtt tattggtgga aattattatt tcaaaacttt tggaggagct    1020
   gtagggaatc acttttatgc aatccaaggg acattggata gtataagtca tcgaccaatt    1080
   ggttttggat taggtgtggg aggaaacgcc tcagcagtat ttacaggggg agaacttgat    1140
   tttactactg gatcagaaac agccttgtta tcatttgtat accaaatagg tgtacaaggt    1200
   gctattgctt taatatgtgt attctacttt atgagtaaag aagtgttgga aaaagtacag    1260
   aagaattcac aattcaaaaa tagattttta ttttatgttc caatgatatt gatttttgtt    1320
   agtatatatc aagctaatac atatacccca caatgtataa cgttgttaat gattactttg    1380
   ggaggatttg taggaatgag agacaggaga aggaaaaaat ataatggtta a             1431
<210> 33
   <211> 702
   <212> DNA
   <213> Lactococcus lactis
<400> 33
   atgataccaa aagtaataca ctattgctgg ttcggagggc aacctttacc agaatctgcg       60
   ctaaaatgta ttgaaagttg gagaaggttt tgtccagatt atgaaataaa acaatggtct      120
   gagaaaaact atgatgtaaa taaaattcaa tatacaaaag aagcgtacga aaagaaacgt      180
   tatgcttttg tcacggatgt tgcaaggctt gatatcattt atagtgaggg gggcatttat      240
   cttgacactg atgttgaact gataaaacct ttggatgatt tgcttgtcaa tcaagcatat      300
   atgggaatgg aaactgctgg tcgtgtaaat accggccaag gatttggagc tataaaaaac      360
   caccaaatag ttaaagcaaa tttagaggtt tatgctgaac ttgagtttga tggtacgact      420
   acttgtgtga cttacacaac taatttgtta aaaagatttg gtttacaaaa aaaagatatt      480
   aaccaggatt tacaaggaat aactatcctt ccgacagatt atttatgtcc attaagtctt      540
   gaaactagga aattagtaat tacagaaaat acattttcta ttcatcacta tgatggtggt      600
   tggaaaaaag gaaaagataa atttttatct ctaaaaatta aaattcgacg ctggattggt      660
   gataactttt atgagtcgat aaaaacgaag ttgaagggat ag                         702

## Description

### FIELD

The present application generally relates to processed cheese compositions and methods for manufacture, and more particularly, processed cheese compositions containing natural antibacterial and antimycotic components.

### BACKGROUND

Processed cheese, widely available in sliced and loaf forms, has become one of the more popular selling cheese products. Processed cheese products are particularly popular with children. Processed cheese is conventionally prepared by heating, grinding and/or mixing one or more varieties of milk-fat containing natural cheeses, such as, for example, Cheddar cheese, Colby cheese, Swiss cheese, Brick cheese, Muenster cheese, pasta filata cheese, washed curd, and granular curd cheese to suggest but a few types. The resulting cheese is then blended with other dairy products, such as non-fat dry milk and whey solids, and emulsifying salts, such as disodium phosphate, at temperatures which are sufficiently elevated to pasteurize the cheese and to produce a homogeneous, pumpable, fluid cheese material that may be formed into sheets, slices, or other desired forms.

It is often desirable to prolong the shelf life of food, such as processed cheese, and/or increase microbiological stability of such food. By increasing the amount of time a food is stable, processors can mitigate inventory losses due to spoiled foodstuffs. Prior methods, such as the use of packaging, preservatives, and/or specific storage parameters (e.g., refrigeration), have been used to stave off spoilage.

In particular, *Listeria monocytogenes* and *C*. *botulinum* can, in some instances, be a concern with foods like as raw milk, cheeses (particularly soft-ripened varieties), ice cream, raw vegetables, fermented raw meat sausages, raw and cooked poultry, raw meats (of all types), and raw and smoked fish. The ability of these pathogens to grow, in some instances, at temperatures as low as 3°C permits multiplication in refrigerated foods.

Furthermore, while it is desired to provide improved shelf life to foods, such as processed cheese, there also has been an increased desire to provide foods that contain an increased amount of natural ingredients. In this regard, it may be desirable to provide foods which include only natural ingredients or otherwise remove artificial materials. For example, processed cheese oftentimes utilizes preservatives such as sorbic acid to improve food safety and shelf life. It may be desirable to incorporate natural preservatives and/or antimicrobials while maintaining and/or improving the characteristics of the processed cheese.

US 2011053832 relates to a natural antimicrobial for foods, comprising a nisin component in the range of 2-40 ppm nisin; and an undissociated organic acid/salt. US 4584199 relates to a pasteurized process cheese product containing at least 54% by weight of moisture containing an amount of nisin or a nisin-producing culture sufficient to inhibit botulinum spore growth. US 5895680 relates to a foodstuff having a pH of about 2.0 to 7.0 and containing a sufficient amount of Natamycin in combination with Nisin to prevent microbial spoilage, wherein the Natamycin is present in an amount of at least about 20 to 500 ppm and said Nisin is present in an amount of about 1 to 50 ppm based on the water content of the foodstuff.

### SUMMARY

Provided herein are processed cheeses including natural antibacterial and antimycotic components. The natural antibacterial is nisin. In some approaches, the nisin includes nisin A. The natural antimycotic is natamycin. It has been unexpectedly found that a processed cheese including nisin and natamycin has a remarkably long shelf-life due to inhibition of growth of mold and Gram- positive microorganisms despite inclusion of low amounts of the nisin and natamycin. It is particularly surprising that these low amounts of nisin and natamycin are effective to prolong the shelf life of the processed cheese products because natamycin is known to be prone to degradation.

In some approaches, the processed cheese is free of artificial preservatives selected from the group consisting of sorbic acid, potassium sorbate, nitrites, and combinations thereof. As used herein, the phrases "does not contain," "is free of" or "substantially free of" mean less than about 0.5 percent, in other approaches, less than about 0.1 percent and, in some cases, less than about 0.05 percent and in other cases, none.

The processed cheese includes an amount of natural antibacterial effective to prevent toxin formation from *Clostridium botulinum* for at least about 9 days when the processed cheese is stored at about 30°C (86°F). The processed cheese may also include natural antibacterial in an amount effective to prevent more than 1 log of growth of *Listeria monocytogenes* for at least about one month, in another aspect at least about two months, in another aspect at least about three months, in another aspect at least about four months, in another aspect at least about five months, in another aspect at least about six months, and in yet
another aspect at least about 7 months, during storage at 7.2°C (45°F). In some aspects, the nisin is nisin A.

The amount of natamycin added during the manufacture of the processed cheese should be selected keeping in mind that a portion of the natamycin will be degraded during the cooking process. Therefore, the amount of natamycin initially added to the processed cheese may be significantly higher than that remaining in the product at, for example, one month, two months, or three months of storage. Generally, it is desirable to select process conditions that optimize the retention of natamycin in the product. It was also found to be desirable to select an initial natamycin concentration that is effective to provide a processed cheese product that, after the cooking step in the process for producing the processed cheese, has retained 0.5 to 6 ppm natamycin, in other approaches about 2 to 6 ppm natamycin, in other approaches about 2 to about 5 ppm natamycin, and in other approaches about 2 to about 4 ppm natamycin, as detected by HPLC. In one aspect, the amount of natamycin may be measured within 72 hours of completion of the cooking step, such as within 72 hours of packaging of the product. As this is the amount remaining after the cooking step, it may be necessary that a larger quantity of natamycin be included initially such that the described quantities remain after at least a portion of the natamycin is degraded during production of the processed cheese.

In some approaches, the processed cheese includes an amount of natural antimycotic effective to prevent mold growth for at least about three months, in another aspect at least about four months, in another aspect at least about five months, in another aspect at least about six months, and in yet another aspect at least about seven months, when the processed cheese is stored at about 1 to about 10°C. The efficacy of the natamycin in the processed cheese product can be determined by conducting a mold challenge study, where samples of the processed cheese are inoculated with a known quantity of mold spores, such as 90 cfu/g or 500 cfu/g, and inspecting the samples for visible mold growth during storage at 4.4 to 7.2°C (40 to 45°F).

The nisin of the processed cheese is included in the processed cheese in the form of a cultured dairy component, which is provided in 1 to 20 percent in the processed cheese. The nisin in the cultured dairy component may also be obtained from a fermentation of a single bacterial strain in a liquid dairy medium. The bacterial strain may be any nisin-producing bacterial strains, such as nisin-producing strains of *Lactococcus lactis.* A preferred *Lactococcus lactis* strain for use herein has all of the identifying characteristics of the *Lactococcus lactis* strain of ATCC PTA-120552.

The processed cheese includes 10 to 90 percent natural cheese or a mixture of natural cheeses; one or more optional emulsifiers; 8 to 25 percent protein; and 10 to 30 percent fat.

A method is also provided for producing a processed cheese including natural antibacterial and antimycotic components. The method includes fermenting a liquid dairy medium with a *Lactococcus lactis* strain to produce a cultured dairy component including nisin and adding natamycin and the cultured dairy component to a natural cheese or mixture of natural cheeses with one or more emulsifiers to produce a processed cheese having 8 to 25 percent protein and 10 to 20 percent fat. The processed cheese includes 0.5 to 6 ppm natamycin and an amount of nisin effective to prevent toxin formation from C. *botulinum* determined by toxin bioassay with mice in the processed cheese at the protein and the fat levels thereof for about 9 days at 30°C (86°F), wherein the processed cheese includes 1 to 100 ppm nisin.

The method may include the fermentation of the *Lactococcus lactis* strain ATCC PTA-120552 conducted in a 2X to a 5X concentrated liquid dairy medium at a temperature of about 25 to about 35°C and a pH of about 5 to about 6 for about 15 to about 48 hours. In some approaches, the concentrated liquid dairy medium is a concentrated milk having a total solids of about 5 to about 36 percent, about 1 to about 14 percent protein, and about 0 to about 16 percent fat.

In other approaches, the method is effective so that the processed cheese is free of artificial preservatives selected from the group consisting of sorbic acid, potassium sorbate, nitrites, and mixtures thereof.

In some approaches, the cultured dairy component of the method includes nisin A and a bacterial strain having at least one gene from a nisin producing gene cluster with significant homology to the sequences selected from the group consisting of SEQ ID NOS 9 to 19.

### BRIEF DESCRIPTION OF THE DRAWINGS

FIG. 1 is a process flow diagram illustrating production of an exemplary cultured dairy component produced from a concentrated dairy liquid;
FIG. 1A is an alternative process flow diagram;
FIG. 2 is a process flow diagram illustrating a second form of production of an exemplary cultured dairy component produced from powdered dairy ingredients;
FIG. 3 is a table showing the results of a mold challenge study of processed cheese including nisin and natamycin;
FIG. 4 is a chart showing the results of phage typing analysis of nisin-producing strains;
FIG. 5 includes results of Riboprinter analysis of various *Lactococcus lactis* strains;
FIG. 6 is a chart comparing the EPS-related genes of various lactic acid bacteria; and
FIG. 7 shows the amino acid sequence of nisin A.

### DETAILED DESCRIPTION

The present invention, which is defined by the claims, is generally directed to processed cheese including, among other aspects, natural antibacterial and antimycotic components. The processed cheese provided herein including both natural antibacterial (nisin) and antimycotic (a polyene compound, natamycin) has significantly improved antimicrobial properties as compared to processed cheese with the same formulation except artificial preservatives and/or other types of prior natural antimicrobials. It was unexpectedly discovered that the combination of natural antibacterial and antimycotic components in the processed cheese described herein was effective inhibit Gram-positive microorganisms such as C. *botulinum* and mold growth at remarkably low levels of both components. In one aspect, the natural antibacterial and natural antimycotic components are incorporated into the processed cheese during production of the cheese and not included as a topical application.

As discussed more below, prior antimicrobials tended to be less effective in the context of a processed cheese with high levels of protein and fat because it was believed that the levels of protein and fat in processed cheese in combination with lower moisture levels provided a cheese matrix that tended to protect and/or shield various pathogens from being inhibited by commercial forms of nisin and other natural antimicrobials. It has been unexpectedly found that a processed cheese including nisin and natamycin has a remarkably long shelf-life due to inhibition of growth of mold and Gram-positive microorganisms despite inclusion of low amounts of the nisin and natamycin. It is particularly surprising that these low amounts of nisin and natamycin are effective to prolong the shelf life of the processed cheese products because natamycin is known to be prone to degradation.

As used herein, the terms "natural antibacterial" and "natural antimycotic" refer to components with antibacterial and antimycotic activities, respectively, which are produced by an organism, such as by a bacterial culture during a fermentation process. One or more different natural antibacterial and antimycotic components may be included in the processed cheese. In one form, the processed cheese contains a sufficient amount of natural antibacterial and antimycotic components such that the processed cheese does not contain or is free of artificial preservatives, such as sorbic acid, potassium sorbate and the like. As used herein, the phrases "does not contain," "is free of" or "substantially free of" mean less than about 0.5 percent, in other approaches, less than about 0.1 percent and, in some cases, less than about 0.05 percent and in other cases, none.

The processed cheese includes an amount of natural antibacterial effective to prevent toxin formation from *Clostridium botulinum* for at least about 9 days when the processed cheese is stored at 30°C (86°F). The processed cheese may also include natural antibacterial in an amount effective to prevent more than 1 log of growth of *Listeria monocytogenes* for at least about one month, in another aspect at least about two months, in another aspect at least about three months, in another aspect at least about four months, in another aspect at least about five months, in another aspect at least about six months, and in yet another aspect at least about seven months, during storage at about 7.2°C (45°F).

The natural antimycotics include polyene antimycotics, and is natamycin, and the natural antibacterial is nisin.
The processed cheeses herein include 1 to 100 ppm nisin, in other approaches about 0.1 to about 20 ppm nisin, in other approaches about 1 to about 15 ppm nisin, in other approaches about 1 to about 10 ppm of nisin, in other approaches about 1 to about 5 ppm of nisin, in other approaches about 3 to about 5 ppm of nisin, and in yet other approaches about 3 to about 4 ppm of nisin. In yet other approaches, the nisin includes nisin A.

The processed cheeses described herein include 0.5 to 6 ppm natural antimycotic, in other approaches about 2 to about 4 ppm natural antimycotic, and in yet other approaches about 2 to about 4 ppm natural antimycotic. The natural antimycotic is natamycin.

The amount of natamycin added during the manufacture of the processed cheese should be selected keeping in mind that a portion of the natamycin will be degraded during the cooking process. Therefore, the amount of natamycin initially added to the processed cheese may be significantly higher than that remaining in the product at, for example, one month, two months, or three months of storage. Generally, it is desirable to select process conditions that optimize the retention of natamycin in the product. It was also found to be desirable to select an initial natamycin concentration that is effective to provide a processed cheese product that, after the cooking step in the process for producing the processed cheese, has retained 0.5 to 6 ppm natamycin, in other approaches about 2 to 6 ppm natamycin, in other approaches about 2 to about 5 ppm natamycin, and in other approaches about 2 to about 4 ppm natamycin, as detected by HPLC. In one aspect, the amount of natamycin may be measured within 72 hours of completion of the cooking step, such as within 72 hours of packaging of the product. As this is the amount remaining after the cooking step, it may be necessary that a larger quantity of
natamycin be included initially such that the described quantities remain after at least a portion of the natamycin is degraded during production of the processed cheese.

In some approaches, the processed cheese includes an amount of natural antimycotic effective to prevent mold growth for at least about three months, in another aspect at least about four months, in another aspect at least about five months, in another aspect at least about six months, and in yet another aspect at least about seven months, when the processed cheese is stored at about 1 to about 10°C. The efficacy of the natamycin in the processed cheese product can be determined by conducting a mold challenge study, where samples of the processed cheese are inoculated with a known quantity of mold spores, such as 90 cfu/ g or 500 cfu/ g, and inspecting the samples for visible mold growth during storage at 4.4 to 7.2°C (40 to 45°F).

The natural antimycotic comprises natamycin, also known as pimaricin. Natamycin can be produced by fermentation using *Streptomyces natalensis* or other organism that has been genetically modified to produce natamycin. Commercially available sources of natamycin can be used, if desired, such as NATAMAX™ SF (87% powdered natamycin) from Danisco. Other natural antifungal agents such as, for example, polylysine (produced by certain *Streptomyces* species) may also be included, if desired.

The natural antibacterial component is incorporated into the processed cheese via cultured dairy components or concentrated cultured dairy components, which include a natural antibacterial component and/or a culture capable of producing a natural antimicrobial under appropriate fermentation conditions. As used herein, the terms "cultured dairy component" or "concentrated cultured dairy component" generally refer to cultured milk substrates or derivatives thereof that have undergone, in some approaches, concentration and fermentation with selected antimicrobial-producing cultures under conditions effective to produce the antibacterial peptides, which is nisin, unless specifically identified as not including cultured antibacterial components.

The natural antibacterial component can be produced by fermentation using an antibacterial-producing strain of lactic acid bacteria. As used herein, the term "lactic acid bacteria" generally refers to gram-positive bacteria that generate lactic acid as a major metabolite of carbohydrate fermentation. The lactic acid bacteria may be, for example, an antibacterial producing strain of *Lactococcus lactis* or, in alternative approaches, *Brevibacterium linens.*

The natural antibacterial component is an antibacterial peptide. The antibacterial peptide is nisin and, in some approaches, may be nisin A. Nisin is an inhibitory polycyclic peptide with 34 amino acid residues. It contains the uncommon amino acids lanthionine, methyllanthionine, dehydroalanine and dehydro-amino-butyric acid. These amino acids are synthesized by posttranslational modifications. In these reactions a ribosomally synthesized 57-mer is converted to the final peptide. The unsaturated amino acids originate from serine and threonine.

Nisin can be obtained by culturing nisin-producing bacteria on natural substrates, including milk. Nisin has been included in food products to extend the safe, usable life by suppressing gram-positive spoilage and pathogenic bacteria. Due to its highly selective activity, it may also be employed as a selective agent in microbiological media for the isolation of gram-negative bacteria, yeast and molds. Two commercially available antimicrobials containing nisin are Nisaplin® and Novasin™ (both from Daniso A/S, Denmark). Typically, Nisaplin contains less than about 3.0 weight % nisin, the remainder consisting of NaCl, proteins, carbohydrates and moisture. When referring to a nisin component herein the component may include not only nisin, but also other ingredients, such as carriers, salts, protein, carbohydrates, and metabolites that result from the fermentation process.

In one aspect, the antibacterial component includes nisin A. Nisin A has a molecular weight of about 3,351.5 Da and the amino acid sequence of SEQ ID NO 1. It should be understood that other natural antimicrobials may also be utilized in the processed cheese products described herein. For example, other forms of nisin, including Nisin Z, Nisin Q, Nisin F, and Nisin F, may be included. Other bacteriocins may also be included, such as class I bacteriocins, class II bacteriocins, class III bacteriocins, and class IV bacteriocins, provided that they are a form of nisin.

Further, bacterial strains that produce natural antimicrobials may be provided. Such bacterial strains include, for example, antibacterial-producing strains of lactic acid bacteria, such as for example, nisin-producing strains of *Lactococcus lactis* or *Brevibacterium linens.*

### Processed Cheese

In one approach, the processed cheese may be produced by blending together natural cheese or mixture of natural cheeses, moisture (e.g., in the form of water or ultra-filtered milk), and an optional additional dairy protein source (such as milk protein concentrate, whey, whey protein concentrate, ultra-filtered milk, and the like). The natural antimycotic, which is natamycin, can also be added at this time. Sodium chloride may be added for flavor. Other optional ingredients may be added to improve texture, flavor, nutrition, and/or cost attributes. These include, but are not limited to, whey derived ingredients (e.g., whey protein concentrate), non-fat dry milk, milk protein concentrate, anhydrous milk fat, gums, starches, gelatin, and the like. The ingredients are blended together and transferred to a cooker (e.g., a laydown cooker) and heated to pasteurization temperatures. The nisin, which is in the form of a cultured dairy component, is added to the cooker. It may be desirable to add the nisin to the cooker, and not prior to the cooking step, because the nisin could be at least partially consumed, thereby decreasing its efficacy during storage, by any microorganism present after the blending step. Optionally, shear may be applied during or after the heating to form a substantially homogenous mass. The product is then packaged in suitable or desired forms, such as slice or loaf form.

Moisture may be added to the blend by any method, such as, but not limited to, injecting steam into the cooker, comingling of condensed steam from cooking, and/or direct addition of water. Of course, moisture can also enter into the system through the various ingredients (e.g., moisture from the natural cheese). Overall moisture of the final cheese products includes all moisture independent of how the moisture was introduced into the final product. Advantageously, because the cultured dairy component includes nisin, water management of the processed cheese is improved. To this end, because nisin and other texture modifying ingredients do not need to be separately added, less water tends to be added into the processed cheese via the cultured dairy component ingredient.

Whey protein refers to a collection of globular proteins that can be isolated from whey, which is the liquid remaining after milk has been curdled and strained. Whey protein is typically a mixture of beta-lactoglobulin, alpha-lactalbumin, and serum albumin proteins. In one embodiment, whey protein concentrate (WPC) may be used as the whey protein source. WPC is derived from whey by conventional concentration techniques. The whey protein source may also include lactose, vitamins, minerals, and fat.

As is known by one of ordinary skill in the art, the ingredients may be used in varying amounts in the processed cheese depending on the desired outcome of the cheese product. For example, for a reduced sodium cheese product, a cheese-maker may include a small amount or no salt in the cheese blend. The processed cheese may also include a range of amounts of the cultured dairy components, depending on the form and composition of the cultured dairy components and the desired form of the processed cheese.

The processed cheese includes 10 to 90% natural cheese. According to another form, the processed cheese may include about 30 to about 60% natural cheese. As used herein, natural cheese refers to unpasteurized cheese made by curdling milk or other dairy liquid using some combination of rennet (or rennet substitute) and acidification. The natural cheese used in the processed cheese described herein may be freshly made or aged.

In yet another form, the processed cheeses herein may include about 35 to about 55% natural cheese. As used herein, natural cheese generally means cheese provided from unpasteurized cheese obtained from curdled milk and one of rennet, rennet substitutes, acidification, and combinations thereof.

The processed cheese may also include a number of other dairy ingredients from various sources as needed for a particular application. For example and in one form, the processed cheese may include milk protein concentrate from about 0 to about 50% (in other approaches, about 10 to about 25%), whey protein concentrate from about 0 to about 25% (in other approaches, about 1 to about 10%), whey from about 0 to about 30% (in other approaches, about 1 to about 10%), milkfat/cream from about 0 to about 30% (in other approaches, about 1 to about 15%) and the like. The processed cheese may also include emulsifiers, such as sodium citrate, disodium phosphate and the like in an amount of about 0% to about 5% (in other approaches, about 1 to about 3%). The processed cheese may also include salt, flavorings, fortifications, colorants and the like to provide the desired color, flavor, etc. The processed cheese may also include added water and/ or moisture from the ingredients to provide the desired finished product moisture. For example, vitamins and other minerals may also be added as needed to fortify the processed cheese, by one approach, from about 0 to about 3 percent of vitamin A, vitamin D and/or calcium powders (such as tricalcium phosphate). In other applications, salt may also be added as needed. In some approaches, about 0 to about 5 percent salt may be added.

Instead of traditional preservatives, the processed cheese includes natamycin and a cultured dairy component containing nisin. In one aspect, the cultured dairy component containing nisin may be made via the methods described herein. The processed cheese includes 1 to 20% cultured dairy component. In another form, the processed cheese includes about 4 to about 10% cultured dairy component. In some approaches, the cultured dairy components of the present disclosure provide a much higher total antimicrobial activity as nisin equivalent relative to the nisin content (a nisin activity ratio). For instance and in some approaches, the cultured dairy components and processed cheese utilizing the amounts of cultured dairy components described herein exhibit a nisin activity ratio of about 0.3 or less.

It should be noted that the natural antibacterial and natural antimycotic components may be used as a replacement for artificial preservatives. When the processed cheeses include the cultured dairy components herein, the cheese may be substantially free of traditional preservatives, such as sorbic acid, nitrites and the like. By one approach, substantially free of generally means less than about 0.5 percent, in other approaches, less than about 0.1 percent, and in other approaches, not present at all.

The natural antibacterial and/or natural antimycotic components also may be used to at least partially supplement or replace other components in the overall processed cheese composition. For example, depending on the form of the cultured dairy component, the amount of the cultured dairy component may be used to supplement or otherwise replace a portion of the other dairy materials in the composition, such as the milk fat, casein, whey and the like. In other words, the ratio of the other dairy materials may be modified as a result of the use of the cultured dairy components.

In one form, the processed cheese includes about 40% natural cheese, 35% other dairy materials, about 8% cultured dairy components, about 12% water and the remainder salts, flavoring, colors, vitamins, minerals and the like. The processed cheese may be manufactured as generally understood with the addition of the cultured dairy components during cooking, and alternatively, during cheese blending steps. In one form, the cheese product described herein may be any of a cheese dip, a cheese spread, a cheese block, a cheese slice, a shredded cheese, or the like. In some approaches, the various forms of processed cheese of the present disclosure may include about 10 to about 90% natural cheese, about 0 to about 50% milk protein concentrate, about 0 to about 30% milk fat or cream, about 40 to about 60% water, 1 to 20% cultured dairy component,, about 0 to about 30% whey, and about 0 to about 25% whey protein concentrate in combination with various optional flavors, salts, and emulsifiers described above.

In another form, the processed cheese includes 10 to 30% total fat (in other approaches, about 20 to 30% fat), 8 to 25% total protein (in other approaches, about 15 to about 25% total protein), and about 40 to about 60% total moisture (in other approaches, about 40 to about 50% moisture).

While not wishing to be limited by theory, it is believed that the cheese matrix provided by the high protein, high fat, and, in some cases, the lower moisture level of the processed cheeses described herein tend to protect and/or encapsulate the natamycin so that it better survives the processed cheese cooking step. That such a low amount of natamycin could be included in the processed cheese and still provide significantly long shelf life without development of mold was surprising and unexpected.

While also not wishing to be limited by theory, it presently appears that the cultured dairy component may play a role in the protection of the natamycin during the cook step. It has been noted that processed cheese products prepared with both natamycin and a cultured dairy component provided by the methods described herein alter the quantity of natamycin detectable by HPLC during storage of the product. For instance, the detectable quantity of natamycin was found to increase in months 2 and 3 of storage and then decrease in month 4. It is presently believed that the cheese matrix changes over time during storage, thereby releasing the natamycin in months 2 and 3 so that it can come into contact with and inactivate mold present in the product. The content of natamycin then decreases in the fourth month as the natamycin is consumed by the mold. The increase in detectable content of natamycin during the second and third months was quite surprising as it was instead expected that the natamycin would degrade over time. This effect was not observed when nisin and natamycin were included in other cheese products, such as cream cheese products. Therefore, it is presently believed that the unique protein and fat matrix of the processed cheese product, and possibly that provided when the cultured dairy component described herein is included in the processed cheese product, surprisingly allows for such low quantities of nisin and natamycin to be included while still providing desired microbial and shelf stability.

The processed cheese made with the cultured dairy components of the present disclosure also exhibits improved antimicrobial characteristics in the context of a high protein and high fat product, such as a processed cheese with about 10 to about 30% fat and about 8 to about 25% protein. While prior commercial forms of nisin were commonly understood to inhibit C. *botulinum* in liquid media or broth, when such prior forms of nisin were used in a high-protein and high-fat food systems, such as processed cheese, the nisin was less effective at inhibiting the C. *botulinum* and other pathogens. While not wishing to be limited by theory, it is believed that the high protein, high fat, and in some case, the lower moisture level of the processed cheeses described herein, tend to protect and/or encapsulate the C. *botulinum* and other pathogens rendering traditional nisin and traditional natural antimicrobials less effective. It was unexpectedly discovered that the nisin obtained from the antimicrobial cultures herein, and in particular, strain 329, are effective to inhibit C. *botulinum* and other food-borne pathogens such as *Listeria monocytogenes,* in the context of high fat and high protein processed cheese much better than other forms of nisin as generally shown below in Example 1. The cultured dairy component in processed cheese provides an amount of nisin effective to prevent toxin formation from at least C. *botulinum* as determined by conventional toxin bioassay with mice in the processed cheese at the high protein and the high fat levels described herein for at least about 9 days at about 30°C (86°F). In one approach, the biotoxin assay may be performed in accordance with Haim M. Solomon et al., Bacteriological Analytical Manual, Chapter 17, Clostridium botulinum, January 2001, available at http://www.fda.gov/Food/FoodScienceResearch/LaboratoryMethods/ucm070879.htm, which is hereby incorporated by reference in its entirety.

Not only are the natamycin and cultured dairy components effective to inhibit mold, C. *botulinum* and other pathogens in the context of a high protein and high fat processed cheese, the cultured dairy components achieve such inhibitory effects at lower activity levels and/or lower dosage levels than previously found possible.

In other approaches, liquid forms of the cultured dairy components herein or made by the processes described herein retain higher levels of nisin activity in the final processed cheese, which was not achievable using prior commercial forms of nisin when used in processed cheeses. By one approach, the cultured dairy ingredients described herein and made by the methods described herein are effective to retain about 50 to about 90 percent activity, and in other approaches, about 60 to about 75 percent activity as compared to the activity of the ingredient prior to incorporation into the processed cheese.

In some approaches, the cultured dairy component is made using an ultrafiltered dairy liquid either before or after fermentation. In these approaches, the cultured dairy component has reduced levels of lactose and other dairy minerals. For example and in some approaches, the cultured dairy component and the processed cheese utilizing the cultured dairy component may have less than about 0.1 percent lactose and less than about 15 percent lactate as acid. In other approaches, the cultured dairy component and the processed cheese utilizing the cultured dairy component may also have less than about 600 mg/100 grams of calcium.

### Production of Antibacterial Component for Use in Processed Cheese

In one form, a cultured dairy component for inclusion in the processed cheese products described herein includes a dairy substrate fermented with an antimicrobial-producing culture. In some approaches, the dairy substrate is a dairy liquid, such as milk or a concentrated dairy liquid or milk substrate, such as a 2-5X concentrated milk substrate. The antimicrobial-producing culture is a nisin-producing culture. In one particular form, the nisin produced by the culture is nisin A.

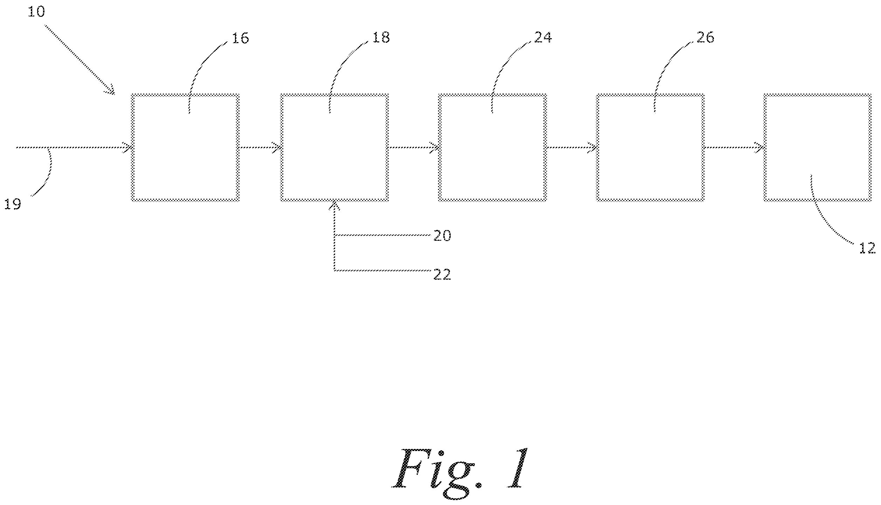

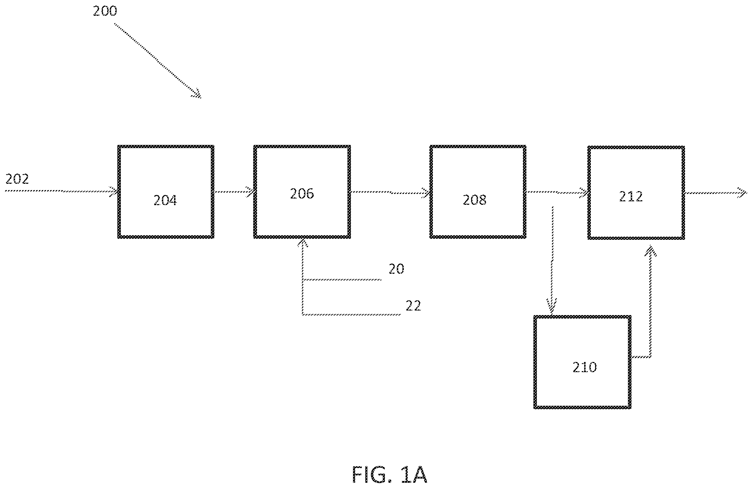

Turning to more of the specifics regarding methods of producing an effective cultured dairy concentrate and processed cheese and first referring to FIG. 1, a process flow diagram 10 is provided that illustrates one method of producing a cultured dairy material or cultured dairy concentrate 12 effective to produce an antibacterial (such as nisin A), the product of which is effective for use with processed cheese. In this exemplary process 10, a liquid dairy starting material 14, such as a dairy liquid like whole milk may be used. In other approaches, the starting dairy liquid may be milk protein concentrate and or whey materials. The starting material 14 may have from about 5 to about 35% total solids, about 0 to about 16 percent fat, about 1 to about 14 percent protein, and about 64 to about 95 percent moisture. In another form, the starting material 14 is 3.5X concentrated whole milk having about 26 to about 30 percent total solids, about 10 to about 15percent fat, about 8 to about 12percent protein, and about 70 to about 70 percent moisture.

In another approach, the starting material 14 is a concentrated dairy liquid obtained from the ultrafiltration of liquid dairy milk. The concentrated starting material may be concentrated to a 2X to a 5X concentration as determined by total solids, in other approaches, about a 2X to about a 4X, and in yet other approaches, about a 3X to about a 3.5X dairy liquid. That is, a 3X concentration has 3 times the level of total solids relative to a starting dairy liquid, and a 5X concentration has about 5 times the level of total solids relative to the starting dairy liquid. In one approach, an ultra-filtration membrane may be used to achieve an appropriate concentrated starting material. One suitable membrane has a molecular weight cutoff of about 5 to about 20 KD. Other methods of concentration may also be used including microfiltration, nanofiltration, and reverse osmosis as needed for a particular application.

As discussed further below, fermentation in concentrated milks, such as the 2X to 5X milk of the present disclosure typically presents problems with prior antimicrobial cultures and fermentations. Strain 329 used within the products and methods of the present disclosure uniquely allows fermentation in a concentrated dairy liquid and, at the same time, permits formation of nisin. By utilizing the concentrated milks for the fermentation and the ultimate production of the processed cheese ingredients herein, water content in the resultant process cheese is better controlled. In some approaches, this reduces the overall moisture load in the processed cheese manufacturing process and, in some cases, also simplifies the processed cheese ingredient line.

The starting material 14, which is either a liquid dairy or concentrated liquid dairy component, is then pasteurized 16 and then enters one or more fermenters 18. Pasteurization may be about 65.6 to about 87.8°C (150 to 190°F) for about 20 to about 40 seconds resulting in an exit temperature of the pasteurized starting material of about 26.7 to about 32.3°C (80 to 90°F). An antimicrobial- producing culture 20, such as *Lactococcus lactis* strain 329, is added to the fermenter 18 along with a base solution 22 such as sodium hydroxide (e.g., a dilute 5.5N sodium hydroxide). In one form, about 2x10⁶ to about 2x10⁸ CFU/ml of the antimicrobial-producing culture 20 is added to the fermenter. In one embodiment, the culture 20 is a thawed form of the culture. In one approach, the fermentation temperature is maintained at about 25 to about 35°C (in some
approaches about 28 to about 32°C, and in other approaches, about 30°C) and a pH of about 5 to about 6 (in other approaches, about 5.4 to about 5.8, and in yet other approaches, about 5.4 to about 5.6) in the fermenter. Other temperatures and pH's may also be used if the culture is able to produce nisin at a desired level under the selected conditions. For example, in one approach, the pH ranges from about 5 to about 7 and the temperature ranges from about 20 to about 40°C. The composition may be fermented over a variety of different time periods to impart different flavor characteristics to the composition. For example, in one approach, the composition is fermented for about 18 to about 22 hours. In another form, the fermentation may take place over a range of about 15 to about 48 hours.

The composition is next sent to an optional shear device 24 to shear small/soft curds that may have formed. In one approach, the shear device may be a rotor/ stator mixer (such as a Dispax) or other rotor shear device. This step may be optional depending on the other processing conditions as well as the properties of the materials utilized in the process. The composition is then finally subjected to an optional heat treatment step 26. In one form, the composition is heat treated 26 at about 65.6 to about 71.1°C (150°F to 160°F) for approximately about 60 to about 100 seconds. In another form, the composition is heat treated to reduce CFU/ ml to less than about 1000 CFU/ ml. The resulting composition 12 is a cultured dairy component or cultured dairy concentrate that includes nisin and/or a nisin-producing material. At least in some approaches, these two components are advantageously produced from the same starting bacterial strain, such as strain 329, and under the same fermentation conditions. The composition may be in the form of a liquid having approximately 6 to about 40% total solids. In one form, the liquid has approximately 20 to about 30% total solids, and in some approaches about 28.5% total solids.

In an alternative method 200 shown in FIG. 1A, hydrated powders and/or liquid milk 202 may be first heated 204, such as in a high temperature, short time sterilization (HTST) or an ultra-high temperature (UHT) sterilization process step. Next, the heated liquid is then fermented 206 with similar materials, cultures, and conditions as described with respect to the previously discussed method. After fermentation, the material may be optionally sheared 208 and then concentrated 210. In this approach, concentration may be membrane filtration, evaporation, or centrifugation. After concentration, the resultant concentrate may be optionally heated 212 again using HTST or UHT, for example.

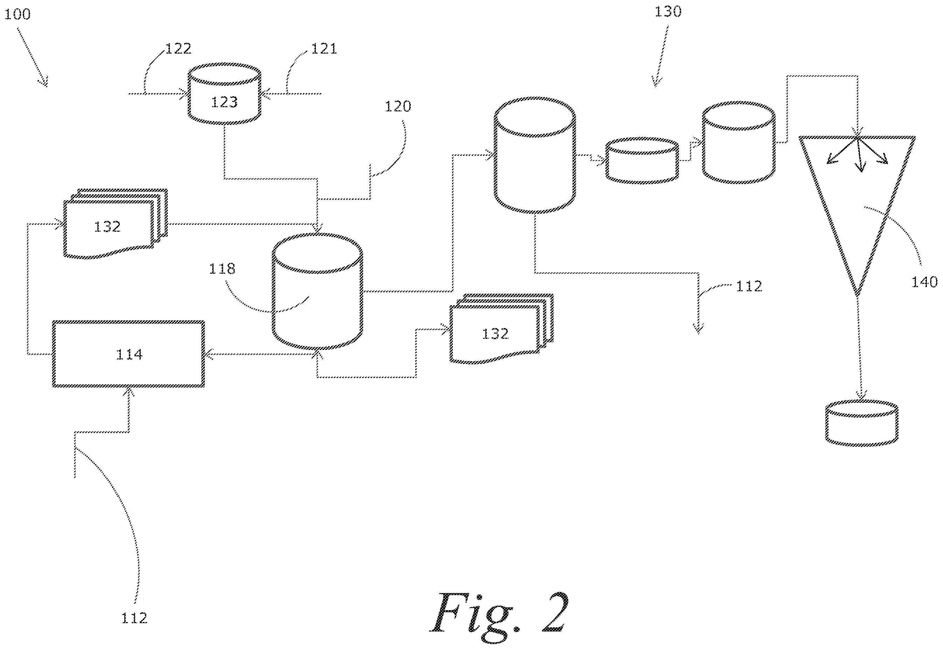

Another process 100 for preparing cultured dairy components is illustrated in FIG. 2. The process 100 in FIG. 2 utilizes powdered starting materials 112 such as powdered milk protein concentrate and powdered whey. In this approach, about 3 to about 10 percent powdered milk protein concentrate, about 2 to about 6 percent powdered whey, and about 75 to about 95 percent water are blended in a tank or a fermenter 118 to form the fermentation medium. The powders may be mixed 114 and hydrated prior to being added to the fermenter 118. These starting materials are then combined with an antimicrobial-producing culture 120, such as *Lactococcus lactis* strain 329, in an amount of about 2x10⁶ CFU/ml to about 2x10⁸ CFU/ml in the fermentation vessel 118 and fermented in a similar manner as described for FIG. 1. Similar to the method of FIG. 1, process water and a base, such as a dilute sodium hydroxide may also be added to the fermentation vessel 118 from tank 123. After fermentation, the composition may optionally be heated or cooled as necessary and then prepared into a final cultured dairy component 112. In some approaches, the process may include various intermediate heating and cooling 132 as needed for a particular application. In this regard, the composition 112 may be placed in one or more holding tanks 130 or other storage location for use in a concentrated liquid form. Holding tank temperatures may be about -1.1 to about 10° C (30 to 50°F). In one embodiment (such as in a liquid form), the cultured dairy component has from about 6 to about 11% total solids and in another form, about 20% total solids. Further, the cultured dairy component may be spray-dried, such as in an atomizer 140. In one approach, the atomizer may have a dryer temperature of about 71.1 to about 82.2°C (160 to 180°F) and about a 103421 to about 172369 Pa (15 to 25 psi) pressure drop.

The cultured dairy component may take a variety of forms. For example, as shown in FIGS. 1 and 2, the cultured dairy component may be in the form of a liquid. The cultured dairy component may also take the form of a powder, such as from spray drying as shown in FIG. 2. Furthermore, the cultured dairy component may also be in a concentrated form, such as components obtained by evaporation, filtering and the like. The resulting product of the method from FIGS. 1 or 2 may be either a liquid or spray dried depending on the particular application. FIG. 2 provides exemplary steps for spray drying and it will be appreciated that these steps can also be used with the methods of FIG. 1. It will be appreciated that if the cultured dairy component is further processed by concentration, spray drying or the like, this further processing will be completed in a manner to not substantially affect the nisin.

The cultured dairy component produced by the methods of FIGS. 1 and 2 may then be used in and/or to manufacture processed cheese.

As described herein, the term activity unit ("AU") may be used to describe the biological activity of the natural antimicrobial in the cultured dairy component and processed cheese in which the antimicrobial is incorporated. It should be understood that biological activity may also be expressed in International Units ("IU") such that AU and IU may be used interchangeably. In some approaches, the cultured dairy components of the present disclosure and the processed cheeses prepared therewith may have nisin activity in the processed cheese of about 40 to about 400 IU/gram and, in other approaches, about 50 to about 200 IU/gram.

In one form, the cultured dairy component comprises a nisin component and/or includes a culture capable of producing a nisin component in the range of about 30 to about 90 ppm by weight of the fermentation medium.

The natural antibacterial can be provided by culturing the antimicrobial-producing bacteria under appropriate fermentation conditions in a dairy substrate. The dairy substrate may include, for example, milk, cream, whey or other dairy-containing powder or liquid. The dairy substrate may also comprise dextrose, corn syrup or other carbohydrates supplemented with other nutrients for bacterial growth, with or without an acid neutralizer such as calcium carbonate.

In some forms, the milk may be in the form of raw milk or a concentrated milk product, such as at least about 2x concentrated milk product, in another aspect up to about 5x concentrated milk product. Typically, the milk base will container greater than about 3 percent lactose and a nitrogen source. The base can be produced from hydrated powders or derived from fresh dairy liquids, such as skim milk, two-percent milk, whole milk, and the like. By one approach, the starting dairy substrate includes concentrated milk having a total solids of about 5 to about 36, a protein content of about 1 to about 14 percent, a fat content of about 0 to about 16 percent, and a moisture content of about 64 to about 95 percent.

When the cultured dairy component is used in production of processed cheese, it has been found to be desirable to maintain a low level of moisture in the dairy substrate to reduce the increased costs associated with removing moisture from the cultured dairy component prior to inclusion in the processed cheese product. Further, certain components of the cultured dairy component may be unstable and may be degraded or otherwise damaged during a moisture removal process. However, the cultured dairy component including cultured antimicrobials may take a variety of forms such as liquid and/or powder, if desired for a particular application.

At least in some approaches, the nisin A-producing culture used herein is *Lactococcus lactis* ss. lactis strain 329. On August 21, 2013, strain 329 was deposited at the American Type Culture Collection (ATCC), 10801 University Blvd., Manassas, VA 20110, and given accession number PTA-120552. The deposit was made under the provisions of the Budapest Treaty on the International Recognition of the Deposit of Microorganisms for the Purposes of Patent Procedure. Strain 329 is also described in detail in U.S. Patent Application No. 13/973,660, filed August 22,2013.

It has been found that *Lactococcus lactis* strain 329 has a unique genetic and phage profile compared to other nisin-producing strains of lactic acid bacteria. Advantageously, strain 329 also was found to be able to grow in concentrated dairy substrates, including about a 2X to about a 5X milk. It has been found that few cultures are capable of growing in such highly concentrated milk substrates. For example, the cultures herein are effective to grow to at least about 10⁹ CFU/gram in about 10 hours and produce more than about 2000IU nisin A/ gram in about 18 hours even in the about 2x to about 5x concentrated fermentation medium. Strain 329 was characterized using Multi Locus Sequence Typing (MLST), phage typing, and ribotyping as discussed more below.

### Multi Locus Sequence Typing (MLST)

The publicly available genome of *L. lactis* subsp. Lactis IL 1403 (taxid:272623), also a nisin-producing strain, was used as a template for selection of seven housekeeping genes to be used as genetic markers in a comparison of IL 1403 to strain 329. The selected genes cover a range of loci on the chromosome as detailed in Table 1 below. Each of the seven genes was amplified and sequenced. Sequences were then aligned and compared using IL1403 as the reference. Each sequence variation was accounted for and represents an individual allele.

**Table 1**

| Gene | Protein | Chromosome Location |
|---|---|---|
| acmD (SEQ ID NO 2) | n-acetylmuramidase | 527,413-528,498 |
| gapB (SEQ ID NO 3) | Glyceraldehyde 3 phosphate dehydrogenase | 2,332,466-2,333,476 |
| pdhD (SEQ ID NO 4) | Lipamidedehydrogenase component of pyruvate dehydrogenase | 58,971-60,389 |
| pepC (SEQ ID NO 5) | Aminopeptidase C | 1,947,325-1,948,635 |
| thiE (SEQ ID NO 6) | Thiamine phosphate pyrophosphorylase | 1,293,610-1,294,257 |
| yjjD (SEQ ID NO 7) | ABC transporter permease protein | 993,341-994,963 |
| yyaL (SEQ ID NO 8) | GTP binding protein | 11,119-12,234 |

### Phage Typing

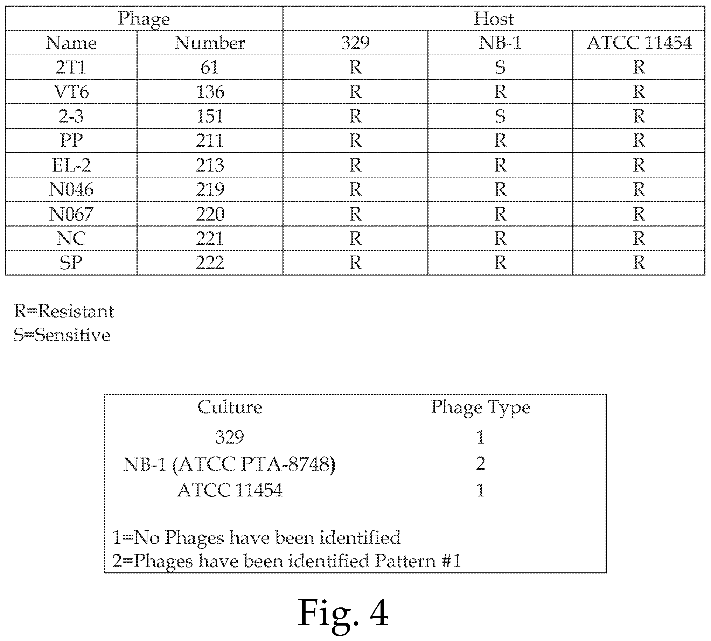

Spot plates were used for phage profiling of high titer phage stocks. The phages are identified and the results of the phage typing are presented in FIG. 4.

### Ribotyping

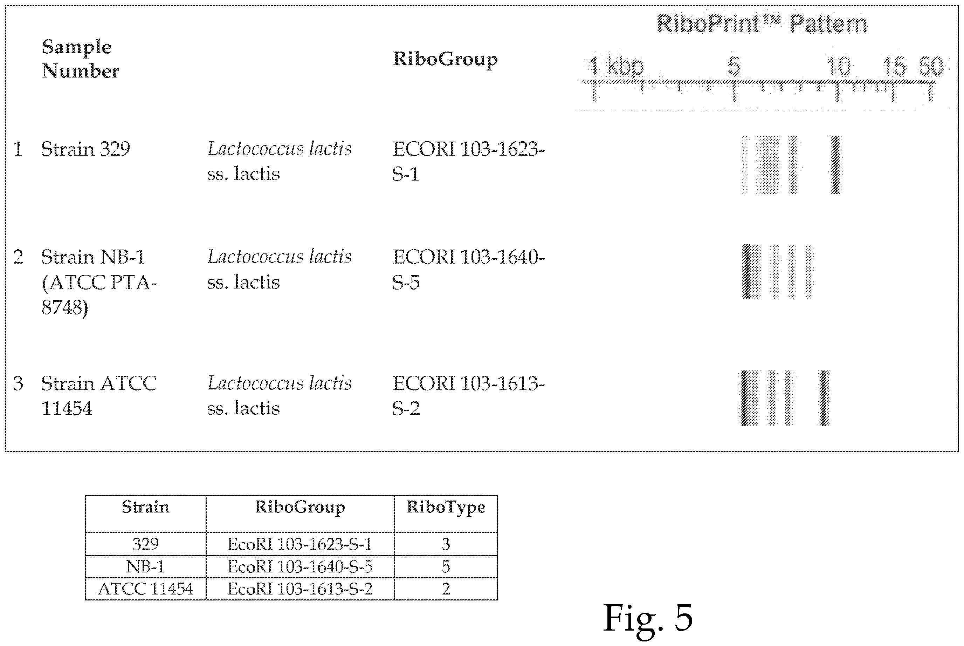

As used herein, "ribotyping" refers to fingerprinting of genomic DNA restriction fragments containing all or part of the genes coding for the 16S and 23S rRNA. Conventional ribotyping techniques utilizing EcoRI as the restriction enzyme were carried out. The results are shown in FIG. 5. Ribotyping confirmed that *Lactococcus lactis* strain 329 is substantially different from publicly available Lactococcus lactis strain ATCC 11454, also a nisin-producing strain.

### DNA Sequence Analysis

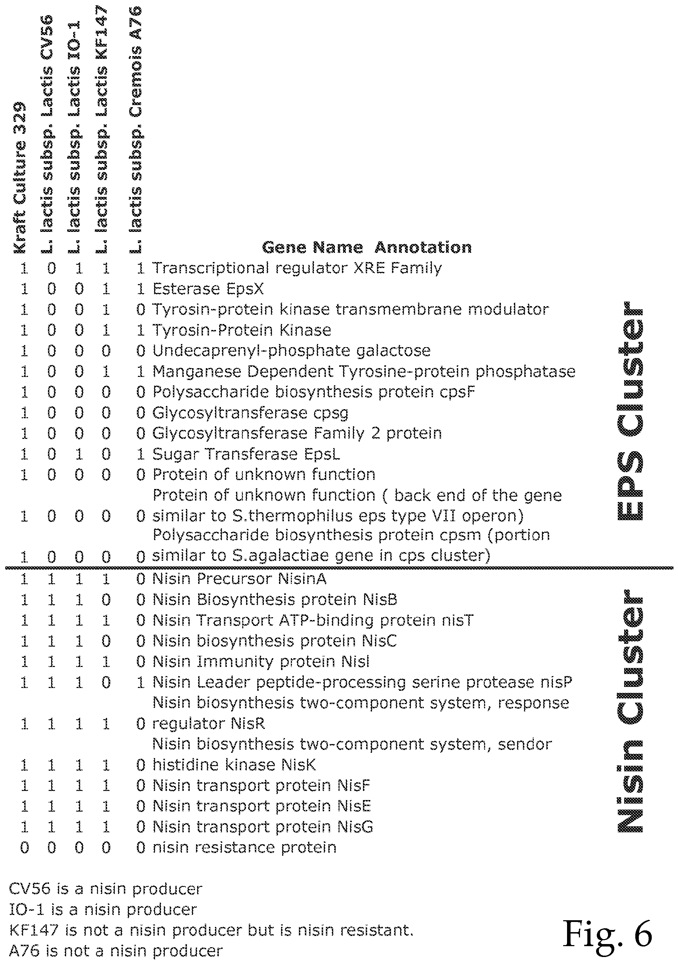

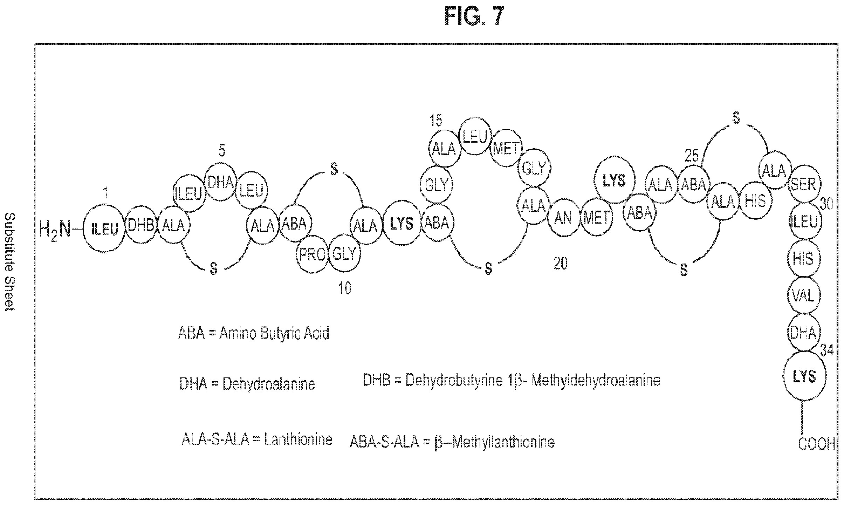

It was found that *Lactococcus lactis* strain 329 has a unique combination of exopolysaccharide and nisin cluster genes as shown in FIG. 6. *Lactococcus lactis* strain 329 includes the nisin cluster genes sequence of Table 2 below and produces nisin A having the amino acid sequence of FIG. 7 (SEQ ID NO 1).

**Table 2**

| GENE | SEQ ID Number |
|---|---|
| Nisin precursor nisin A | SEQ ID NO 9 |
| Nisin transport protein (nisG) | SEQ ID NO 10 |
| Nisin transport protein (nisE) | SEQ ID NO 11 |
| Nisin transport protein (nisF) | SEQ ID NO 12 |
| Nisin two-component system, response regulator (nisR) | SEQ ID NO 13 |
| Nisin sensor-receptor histidine kinase (nisK) | SEQ ID NO 14 |
| Nisin leader peptide-processing serine protease (nisP) | SEQ ID NO 15 |
| Nisin immunity protein | SEQ ID NO 16 |
| Nisin biosynthesis protein (nisC) | SEQ ID NO 17 |
| Nisin transport ATP-binding protein (nisT) | SEQ ID NO 18 |
| Nisin biosynthesis protein (nisB) | SEQ ID NO 19 |

It has been found that, at least under fermentation conditions described herein with reference to the method of FIG. 1, *Lactococcus lactis* strain 329 produces a high level of 34-mer nisin A relative to the 57-mer nisin A precursor peptide (nisin A precursor has the amino acid sequence of SEQ ID NO 20). Nisin A is produced by posttranslational modification of the nisin A precursor. At least in some approaches, *Lactococcus lactis* strain 329 produces nisin A relative to nisin A precursor at a ratio of at least about 9:1, in another aspect at least about 9.5:0.5. In contrast, Danisco's Nisaplin® includes approximately 83 percent nisin A and 17 percent nisin A precursor.

Under the same fermentation conditions effective to produce nisin A described above, *Lactococcus lactis* strain 329 also produces exopolysaccharide, such as, for example, under fermentation conditions described herein with reference to the method of FIG. 1. *Lactococcus lactis* strain 329 includes the EPS cluster genes of Table 3 below:

**Table 3**

| GENE | SEQ ID Number |
|---|---|
| Transcriptional regulator, XRE family | SEQ ID NO 21 |
| Esterase (EpsX) | SEQ ID NO 22 |
| Tyrosine-protein kinase transmembrane modulator (epsC) | SEQ ID NO 23 |
| Tyrosine-protein kinase | SEQ ID NO 24 |
| Undecaprenyl-phosphate galactose phosphotransferase | SEQ ID NO 25 |
| Manganese depended tyrosine-protein phosphatase | SEQ ID NO 26 |
| Polysaccharide biosynthesis protein (cpsF) /glycosyl transferase (cpsG) | SEQ ID NO 27 |
| Glycosyl transferase (cpsG) / polysaccharide biosynthesis protein (cpsM(v)) | SEQ ID NO 28 |
| Glycosyltransferase family 2 protein | SEQ ID NO 29 |
| Sugar transferase, (epsL) | SEQ ID NO 30 |
| Protein of unknown function, unknown family | SEQ ID NO 31 |
| Protein of unknown function, unknown family / Beta-1,3-glucosyltransferase | SEQ ID NO 32 |
| Polysaccharide biosynthesis protein (cpsM(v)) | SEQ ID NO 33 |

At least in some approaches, an antimicrobial-producing bacterial strain useful in the methods described herein is able to produce both nisin A of about 2000 IU/gram or more and exopolysaccharide under the fermentation conditions described in reference to FIG. 1.

The sequence information provided herein should not be so narrowly construed so as to require inclusion of erroneously identified nucleotides. The sequences disclosed herein can be used by one of ordinary skill in the art to isolate the complete gene from the bacterial strain and subject the gene to further sequence analysis to identify any sequencing errors.

As used herein, the terms "homology" and "identity" are used interchangeably. For purposes of determining the percent identity or homology of two sequences, the sequences may be aligned for optimal comparison purposes. The nucleotides or amino acids are then compared at corresponding nucleotide or amino acid positions of the two sequences. For example, a nucleotide or amino acid in a first sequence is considered identical to that of the second sequence when the same nucleotide or amino acid is located in the corresponding position in the second sequence. The percent identity is calculated by determining the number of identical positions divided by the total number of positions (i.e., overlapping positions) multiplied by 100.

Functional nucleic acid equivalents are also contemplated herein. For example, functional nucleic acid equivalents include silent mutations or other mutations that do not alter the biological function of the encoded polypeptide.

In one form, an antimicrobial-producing bacterial strain useful in the methods described herein has one or more genes of significant homology to SEQ ID NOS 9-19 and 21-33 and is able to produce nisin A. As defined herein, the term "significant homology" means at least 70 percent, in another aspect at least 75 percent, in another aspect at least 80 percent, in another aspect at least 85 percent identity, in another aspect at least 90 percent identity, in another aspect at least 95 percent identity, in yet another aspect at least 99 percent identity, and in yet another aspect complete identity.

In some approaches, an antimicrobial-producing bacterial strain useful in the methods described herein has at least two genes of significant homology to the sequences selected from the group consisting of SEQ ID NOS 9-19 and 21-33, in another aspect has at least three genes of significant homology to the sequences selected from the group consisting of SEQ ID NOS 9-19 and 21-33, in another aspect has at least four genes of significant homology to the sequences selected from the group consisting of SEQ ID NOS 9-19 and 21-33, in another aspect has at least five genes of significant homology to the sequences selected from the group consisting of SEQ ID NOS 9-19 and 21-33, in another aspect has at least six genes of significant homology to the sequences selected from the group consisting of SEQ ID NOS 9-19 and 21-33, in another aspect has at least seven genes of significant homology to the sequences selected from the group consisting of SEQ ID NOS 9-19 and 21-33, in another aspect has at least eight genes of significant homology to the sequences selected from the group consisting of SEQ ID NOS 9-19 and 21-33, in another aspect has at least nine genes of significant homology to the sequences selected from the group consisting of SEQ ID NOS 9-19 and 21-33, in another aspect has at least ten genes of significant homology to the sequences selected from the group consisting of SEQ ID NOS 9-19 and 21-33, in another aspect has at least eleven genes of significant homology to the sequences selected from the group consisting of SEQ ID NOS 9-19 and 21-33, in another aspect has at least twelve genes of significant homology to the sequences selected from the group consisting of SEQ ID NOS 9-19 and 21-33, in another aspect has at least thirteen genes of significant homology to the sequences selected from the group consisting of SEQ ID NOS 9-19 and 21-33, in another aspect has at least fourteen genes of significant homology to the sequences selected from the group consisting of SEQ ID NOS 9-19 and 21-33, in another aspect has at least fifteen genes of significant homology to the sequences selected from the group consisting of SEQ ID NOS 9-19 and 21-33, in another aspect has at least sixteen genes of significant homology to the sequences selected from the group consisting of SEQ ID NOS 9-19 and 21-33, in another aspect has at least seventeen genes of significant homology to the sequences selected from the group consisting of SEQ ID NOS 9-19 and 21-33, in another aspect has at least eighteen genes of significant homology to the sequences selected from the group consisting of SEQ ID NOS 9-19 and 21-33, in another aspect has at least nineteen genes of significant homology to the sequences selected from the group consisting of SEQ ID NOS 9-19 and 21-33, in another aspect has at least twenty genes of significant homology to the sequences selected from the group consisting of SEQ ID NOS 9-19 and 21-33, in another aspect has at least twenty-one genes of significant homology to the sequences selected from the group consisting of SEQ ID NOS 9-19 and 21-33, in another aspect has at least twenty-two genes of significant homology to the sequences selected from the group consisting of SEQ ID NOS 9-19 and 21-33, in another aspect has at least twenty-three genes of significant homology to the sequences selected from the group consisting of SEQ ID NOS 9-19 and 21-33, and in yet another aspect has at least twenty-four genes of significant homology to the sequences selected from the group consisting of SEQ ID NOS 9-19 and 21-33.

Advantages and embodiments of the compositions, methods, and compositions produces by the methods described herein are further illustrated by the following examples; however, the particular conditions, processing schemes, materials, and amounts thereof recited in these examples, as well as other conditions and details, should not be construed to unduly limit this method. All percentages and ratios within this disclosure are by weight unless otherwise indicated.

### EXAMPLES

The following examples illustrate the performance of processed cheese slices prepared with cultured dairy components as described above and control samples of process cheese without the cultured dairy component but, instead, using sorbic acid as the preservative. The samples were generally prepared as processed cheese slices having about 46 percent moisture, about 23 percent fat, about 1.2 percent salt, and about 18 percent protein with varying amounts of preservatives and/or cultured dairy components (where indicated), flavors, colors, vitamins, minerals and the like.

### Example 1 (comparative)

Natamycin (Natamax SF from Danisco) was incorporated into processed cheese (prior to the cooking step) at concentrations of 7 ppm and 15 ppm. The processed cheese was then submitted to a mold challenge study. The processed cheese was inoculated with a mold cocktail (spores in liquid media) of Phoma spp., Geotrichum spp., Cladosporium spp., and Penicillium spp. (which represent common types of mold) at 90 spores per 19 gram slice. The samples are inoculated by spreading the cocktail on top of cheese slices. A control was not inoculated. Processed cheese containing the natamycin significantly extended shelf life by at least six months longer than a control that lacked mold inhibitor. It was surprisingly found that the cheese containing natamycin was effective to extend the shelf life beyond six months when stored at 7.2°C (45°F) even though the detectable natamycin concentration in the product had decreased to 0.62 ppm (for the 7 ppm sample) and 4.89 ppm (for the 15 ppm sample) by six months. The detectable level of natamycin was determined by HPLC.

### Example 2

Natamycin (Natamax SF from Danisco) was incorporated into processed cheese (prior to the cooking step) at concentrations of 0 ppm (comparative), 10 ppm, and 15 ppm. Four samples of each dosage were prepared. Cultured dairy component containing nisin was incorporated into all products at 8 percent based on the total weight of the processed cheese. When measured three days after producing the processed cheese, the samples starting with 10 ppm and 15 ppm natamycin had, on average, 2.8 ppm and 4.9 ppm natamycin, respectively.

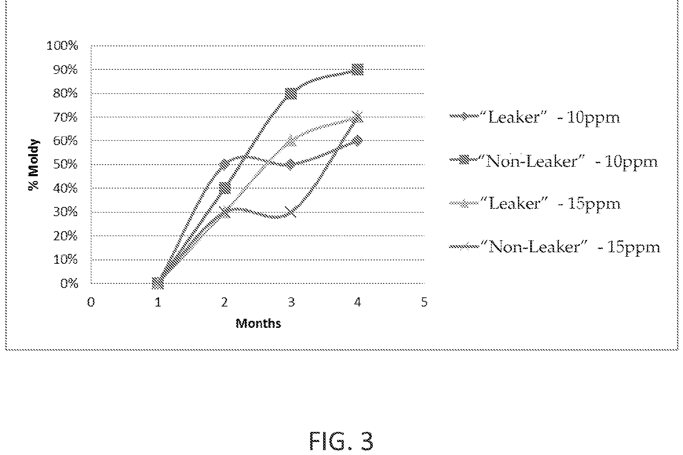

The products were inoculated at 500 cfu/ g or 9500 cfu/ slice with the mold cocktail of Example 1 by applying the cocktail to the surface of the cheese slices and leaving the packages open. Other packages of the processed cheese (not inoculated with the mold cocktail) were intentionally punctured to provide holes in the package to simulate a real world situation. The results of the 9500 spores per slice inoculation (designated "non-leaker") and punctured package experiments (designated "leaker") are shown in FIG. 3. These samples exhibited shelf life of almost double that of control when inoculated with 500 cfu/ g or 9,500 spores per slice of processed cheese, even with the extremely large 9,500 spores/slice inoculation level.

### Example 3 (comparative)

Processed cheese slices were produced containing about 4.8 ppm natamycin (Natamax SF from Danisco). The slices had no visible mold at five months of shelf life when stored at 7.2°C (45°F), whereas a control without natamycin showed visible mold at three months.

### Example 4

Natamycin (Natamax SF from Danisco) was incorporated into processed cheese (prior to the cooking step) at 15 ppm. Cultured dairy component containing nisin was incorporated into all products at 8 percent based on the total weight of the processed cheese. Samples of the processed cheese were inoculated at 100 cfu/ g and 500 cfu/ g. It was found that the detectable level of natamycin changed during storage of the product at 7.2°C (45°F) as shown in Table 4 below. The detectable level of natamycin was determined by HPLC.

**Table 4**

| Natamycin Dosage Level | Detected Natamycin Concentration (ppm) after One Week | Detected Natamycin Concentration (ppm) after Two Months | Detected Natamycin Concentration (ppm) after Three Months | Detected Natamycin Concentration (ppm) after Four Months |
|---|---|---|---|---|
| 15 ppm | 4.63 | 6.81 | 6.78 | 3.28 |
| 15 ppm | 4.80 | 5.67 | 7.36 | 3.14 |
| 15 ppm | 4.61 | 6.73 | 6.87 | 3.07 |
| 15 ppm | - | 4.62 | 6.34 | 3.39 |
| 15 ppm | - | 6.32 | 7.46 | 3.64 |
| 15 ppm | - | 5.34 | 6.13 | 2.97 |
| 15 ppm | - | 5.91 | 6.84 | 3.37 |
| | Average = 4.68 | Average= 5.93 | Average= 6.77 | Average = 3.39 |

At approximately three months, the detectable natamycin level appears to peak to an average of 6.8, which is advantageous from a commercial perspective as processed cheese product may remain available in stores at three months.

## Claims

1. A processed cheese including natural antibacterial and antimycotic components, the processed cheese comprising:
10 to 90 percent natural cheese or a mixture of natural cheeses;
one or more emulsifiers;
8 to 25 percent protein;
10 to 30 percent fat;
1 to 20 percent of a cultured dairy component, the cultured dairy component comprising an amount of nisin effective to prevent toxin formation from *C*. *botulinum* determined by toxin bioassay with mice in the processed cheese at the protein and the fat levels thereof for about 9 days at 30°C (86°F), wherein the processed cheese includes 1 to 100 ppm nisin; and
0.5 to 6 ppm natamycin.

2. The processed cheese of claim 1, wherein the processed cheese includes 2 to 6 ppm natamycin.

3. The processed cheese of claim 1, wherein the processed cheese includes 2 to 5 ppm natamycin.

4. The processed cheese of claim 1, wherein the cultured dairy component is provided by fermentation of an isolated *Lactococcus lactis* strain having all of the identifying characteristics of the *Lactococcus lactis* strain of ATCC PTA-120552.

5. The processed cheese of claim 4, wherein the fermentation of the bacterial strain ATCC PTA-120552 is conducted in a 3X to a 5X concentrated liquid dairy medium at a temperature of 25 to 35°C and a pH of 5 to 6 for 15 to 48 hours.

6. The processed cheese of claim 1, wherein the processed cheese is free of artificial preservatives selected from the group consisting of sorbic acid, potassium sorbate, nitrites, and mixtures thereof.

7. The processed cheese of claim 1, wherein the nisin is nisin A.

8. A method of producing a processed cheese including natural antibacterial and antimycotic components, the method comprising:
fermenting a liquid dairy medium with a *Lactococcus lactis* strain to produce a cultured dairy component including nisin; and
adding natamycin and the cultured dairy component to a natural cheese or mixture of natural cheeses with one or more emulsifiers to produce a processed cheese having 8 to 25 percent protein and 10 to 20 percent fat;
wherein the processed cheese includes 1 to 20 percent of the cultured dairy component, and the processed cheese includes 0.5 to 6 ppm natamycin and an amount of nisin effective to prevent toxin formation from *C*. *botulinum* determined by toxin bioassay with mice in the processed cheese at the protein and the fat levels thereof for about 9 days at 30°C (86°F), wherein the processed cheese includes 1 to 100 ppm nisin.

9. The method of claim 8, wherein the processed cheese includes 2 to 6 ppm natamycin.

10. The method of claim 8, wherein the processed cheese includes 2 to 5 ppm natamycin.

11. The method of claim 8, wherein the cultured dairy component includes 1 to 100 ppm of nisin.

12. The method of claim 8, wherein the processed cheese is free of artificial preservatives selected from the group consisting of sorbic acid, potassium sorbate, nitrites, and mixtures thereof.

13. The method of claim 8, wherein the nisin is nisin A.

## Patentansprüche

1. Verarbeiteter Käse, der natürliche antibakterielle und antimykotische Komponenten beinhaltet, wobei der verarbeitete Käse Folgendes umfasst:
10 bis 90 Prozent Naturkäse oder eine Mischung aus Naturkäsen;
einen oder mehr Emulgatoren;
8 bis 25 Prozent Protein;
10 bis 30 Prozent Fett;
1 bis 20 Prozent einer kultivierten Milchkomponente, wobei die kultivierte Milchkomponente eine Menge Nisin umfasst, die wirksam ist, um eine Toxinbildung von *C*. *botulinum* zu verhindern, was durch einen Toxin-Bioassay mit Mäusen am verarbeiteten Käse bei den Protein- und Fettspiegeln davon über etwa 9 Tage bei 30°C (86°F) ermittelt wird, wobei der verarbeitete Käse 1 bis 100 ppm Nisin beinhaltet; und
0,5 bis 6 ppm Natamycin.

2. Verarbeiteter Käse nach Anspruch 1, wobei der verarbeitete Käse 2 bis 6 ppm Natamycin beinhaltet.

3. Verarbeiteter Käse nach Anspruch 1, wobei der verarbeitete Käse 2 bis 5 ppm Natamycin beinhaltet.

4. Verarbeiteter Käse nach Anspruch 1, wobei die kultivierte Milchkomponente durch Fermentation eines isolierten *Lactococcus lactis-*Stammes bereitgestellt wird, der alle der identifizierenden Charakteristika des *Lactococcus lactis*-Stammes ATCC PTA-120552 aufweist.

5. Verarbeiteter Käse nach Anspruch 4, wobei die Fermentation des Bakterienstammes ATCC PTA-120552 15 bis 48 Stunden lang in einem 3X bis 5X konzentrierten flüssigen Milchmedium bei einer Temperatur von 25 bis 35°C und einem pH-Wert von 5 bis 6 durchgeführt wird.

6. Verarbeiteter Käse nach Anspruch 1, wobei der verarbeitete Käse frei von künstlichen Konservierungsstoffen ist, die ausgewählt sind aus der Gruppe bestehend aus Sorbinsäure, Kaliumsorbat, Nitriten und Mischungen davon.

7. Verarbeiteter Käse nach Anspruch 1, wobei das Nisin Nisin A ist.

8. Verfahren zur Herstellung eines verarbeiteten Käses, der natürliche antibakterielle und antimykotische Komponenten beinhaltet, wobei das Verfahren Folgendes umfasst:
Fermentieren eines flüssigen Milchmediums mit einem *Lactococcus lactis-Stamm,* um eine kultivierte Milchkomponente, die Nisin beinhaltet, herzustellen; und
Hinzufügen von Natamycin und der kultivierten Milchkomponente zu einem Naturkäse oder einer Mischung von Naturkäsen mit einem oder mehreren Emulgatoren, um einen verarbeiteten Käse herzustellen, der 8 bis 25 Prozent Protein und 10 bis 20 Prozent Fett aufweist;
wobei der verarbeitete Käse 1 bis 20 Prozent der kultivierten Milchkomponente beinhaltet und der verarbeitete Käse 0,5 bis 6 ppm Natamycin und eine Menge von Nisin enthält, die wirksam ist, um die Toxinbildung von *C*. *botulinum* zu verhindern, was durch einen Toxin-Bioassay mit Mäusen am verarbeiteten Käse bei den Protein- und Fettspiegeln davon über etwa 9 Tage bei 30°C (86°F) ermittelt wird, wobei der verarbeitete Käse 1 bis 100 ppm Nisin beinhaltet.

9. Verfahren nach Anspruch 8, wobei der verarbeitete Käse 2 bis 6 ppm Natamycin beinhaltet.

10. Verfahren nach Anspruch 8, wobei der verarbeitete Käse 2 bis 5 ppm Natamycin beinhaltet.

11. Verfahren nach Anspruch 8, wobei die kultivierte Milchkomponente 1 bis 100 ppm Nisin beinhaltet.

12. Verfahren nach Anspruch 8, wobei der verarbeitete Käse frei von künstlichen Konservierungsstoffen ist, die ausgewählt sind aus der Gruppe bestehend aus Sorbinsäure, Kaliumsorbat, Nitriten und Mischungen davon.

13. Verfahren nach Anspruch 8, wobei das Nisin Nisin A ist.

## Revendications

1. Fromage à pâte fondue incluant des composants antibactériens et antimycosiques naturels, le fromage à pâte fondue comprenant :
de 10 à 90 pour cent d'un fromage naturel ou d'un mélange de fromages naturels ;
un ou plusieurs émulsifiants ;
de 8 à 25 pour cent de protéines ;
de 10 à 30 pour cent de matières grasses ;
de 1 à 20 pour cent d'un composant laitier cultivé, le composant laitier cultivé comprenant une nisine à une quantité efficace pour empêcher la formation de la toxine de *C. botulinum,* telle que déterminée par un dosage biologique de la toxine chez des souris exposées au fromage à pâte fondue contenant des protéines et des matières grasses aux teneurs indiquées pendant environ 9 jours à 30 °C (86 °F), le fromage à pâte fondue incluant de 1 à 100 ppm d'une nisine ; et
de 0,5 à 6 ppm de natamycine.

2. Fromage à pâte fondue de la revendication 1, où le fromage à pâte fondue inclut de 2 à 6 ppm de natamycine.

3. Fromage à pâte fondue de la revendication 1, où le fromage à pâte fondue inclut de 2 à 5 ppm de natamycine.

4. Fromage à pâte fondue de la revendication 1, où le composant laitier cultivé est obtenu par fermentation d'une souche de *Lactococcus lactis* isolée ayant toutes les caractéristiques identificatrices de la souche ATCC PTA-120552 de *Lactococcus lactis.*

5. Fromage à pâte fondue de la revendication 4, où la fermentation de la souche bactérienne ATCC PTA-120552 est effectuée dans un milieu laitier liquide à 3 à 5 fois concentré à une température qui va de 25 à 35 °C et à un pH qui va de 5 à 6 pendant 15 à 48 heures.

6. Fromage à pâte fondue de la revendication 1, où le fromage à pâte fondue est exempt d'agents conservateurs artificiels sélectionnés dans le groupe consistant en l'acide sorbique, le sorbate de potassium, des nitrites et des mélanges de ceux-ci.

7. Fromage à pâte fondue de la revendication 1, où la nisine est la nisine A.

8. Procédé de production d'un fromage à pâte fondue incluant des composants antibactériens et antimycotiques naturels, le procédé comprenant :
la fermentation d'une souche de *Lactococcus lactis* dans un milieu laitier liquide pour produire un composant laitier cultivé incluant une nisine ; et
l'addition de natamycine et du composant laitier cultivé à un fromage naturel ou à un mélange de fromages naturels avec un ou plusieurs émulsifiants pour produire un fromage à pâte fondue ayant de 8 à 25 pour cent de protéines et de 10 à 20 pour cent de matières grasses ;
où le fromage à pâte fondue inclut de 1 à 20 pour cent du composant laitier cultivé et où le fromage à pâte fondue inclut de 0,5 à 6 ppm de natamycine et une nisine à une quantité efficace pour empêcher la formation de la toxine de *C. botulinum,* telle que déterminée par un dosage biologique de la toxine chez des souris exposées au fromage à pâte fondue contenant des protéines et des matières grasses aux teneurs indiquées pendant environ 9 jours à 30 °C (86 °F), le fromage à pâte fondue incluant de 1 à 100 ppm d'une nisine.

9. Procédé de la revendication 8, où le fromage à pâte fondue inclut de 2 à 6 ppm de natamycine.

10. Procédé de la revendication 8, où le fromage à pâte fondue inclut de 2 à 5 ppm de natamycine.

11. Procédé de la revendication 8, où le composant laitier cultivé inclut de 1 à 100 ppm d'une nisine.

12. Procédé de la revendication 8, où le fromage à pâte fondue est exempt d'agents conservateurs artificiels sélectionnés dans le groupe consistant en l'acide sorbique, le sorbate de potassium, des nitrites et des mélanges de ceux-ci.

13. Procédé de la revendication 8, où la nisine est la nisine A.
